(19) Europäisches Patentamt
European Patent Office
Office européen des brevets

(11) EP 4 026 665 B1

(12) **EUROPEAN PATENT SPECIFICATION**

(45) Date of publication and mention
of the grant of the patent:
**27.08.2025 Bulletin 2025/35**

(21) Application number: **20860585.7**

(22) Date of filing: **10.07.2020**

(51) International Patent Classification (IPC):
**B25J 11/00** (2006.01)    **A61F 2/72** (2006.01)
**A61B 5/107** (2006.01)    **A61B 5/11** (2006.01)
**A61B 5/00** (2006.01)

(52) Cooperative Patent Classification (CPC):
**A61B 5/6831; A61B 5/1114; A61B 5/1116;**
**A61B 5/1126; A61B 5/4561; A61B 5/4566;**
**A61B 5/6811; A61B 5/6835; A61B 5/7275;**
**B25J 9/0006;** A61B 5/1071; A61B 2503/20;
A61B 2505/09; A61B 2562/0219

(86) International application number:
**PCT/JP2020/027170**

(87) International publication number:
**WO 2021/044734 (11.03.2021 Gazette 2021/10)**

(54) **HIP LOAD EVALUATION DEVICE IN WHICH WEARABLE MOVEMENT-ASSIST DEVICE IS USED, AND METHOD FOR EVALUATION OF HIP LOAD**

VORRICHTUNG ZUR BEWERTUNG DER HÜFTBELASTUNG MIT VERWENDUNG EINES AM KÖRPER TRAGBAREN BEWEGUNGSGESTÜTZTEN GERÄTS UND VERFAHREN ZUR BEWERTUNG DER HÜFTBELASTUNG

DISPOSITIF D'ÉVALUATION DE CHARGE DE LA HANCHE DANS LEQUEL EST UTILISÉ UN DISPOSITIF D'ASSISTANCE AU MOUVEMENT POUVANT ÊTRE PORTÉ SUR SOI, ET PROCÉDÉ D'ÉVALUATION DE LA CHARGE DE LA HANCHE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2019 JP 2019159978**

(43) Date of publication of application:
**13.07.2022 Bulletin 2022/28**

(73) Proprietor: **CYBERDYNE INC.**
**Tsukuba-shi, Ibaraki 305-0818 (JP)**

(72) Inventor: **HARA, Hiromasa**
**Ibaraki 305-0818 (JP)**

(74) Representative: **Mewburn Ellis LLP**
**Aurora Building**
**Counterslip**
**Bristol BS1 6BX (GB)**

(56) References cited:
WO-A1-2017/039018    WO-A1-2019/161232
JP-A- 2012 183 291    JP-A- 2013 173 190
JP-A- 2018 015 023    US-A1- 2018 140 245

• HIROSHI FUKUI, TSUNEO KAWANO, YUSUKE TANAKA: "Development of Personal Alarm System for Low Back Pain with Smart-phone", JOURNAL OF MOBILE INTERACTIONS, vol. 3, no. 1, 1 August 2013 (2013-08-01), pages 7 - 12, XP009534953, ISSN: 2185-6796
• KATSUO HASEGAWA: "Workload analysis of wakame seaweed harvesting at Sanriku district.", THE JAPANESE SOCIETY OF FISHERIES ENGINEERING, vol. 43, no. 2, 15 November 2006 (2006-11-15), pages 179 - 184, XP055907957, ISSN: 0916-7617, DOI: 10.18903/fisheng.43.2_179

- **KIMIYASU SAEKI , NOBUO KIMURA, YASUZUMI FUJIMORI, HIROKI YASUMA, SHUJI HISAMUNE: "Estimation of the Compression Force in the Lumbar Intervertebral Disk During the Transportation of Fish Catch in Fishing Ports", THE JAPANESE SOCIETY OF FISHERIES ENGINEERING, vol. 51, no. 2, 15 November 2014 (2014-11-15), pages 133 - 137, XP055907965, ISSN: 0916-7617, DOI: 10.18903/fisheng.51.2_133**

- **DOI, TATSUYA; YAMADA, HIRONAO; IKEMOTO, TOSHIHISA; NARATANI, HIROSHI: "Simulation of a pneumatic hand crane type power assist system", TRANSACTIONS OF THE JAPAN FLUID POWER SYSTEM SOCIETY, vol. 39, no. 2, 31 March 2008 (2008-03-31), JP, pages 28 - 33, XP009534966, ISSN: 1880-3121, DOI: 10.5739/jfps.39.28**

**Description**

TECHNICAL FIELD

The present disclosure relates to a lower back part load evaluation apparatus and lower back part load evaluation method using a wearable motion assistance device and is suited for application to a wearable motion assistance device which assists motions of heavy muscular work by mounting it on a wearer's lower back part. The invention is defined by the appended claims.

BACKGROUND ART

Lower back pain is a disease with high disease prevalence, recurrence rate, and chronicity rate and it is said that 84% of the human race experience at least once in their lives. Conventionally, examples of work and environmental factors attributable to the occurrence of occupational lower back pain include object lifting motions, retaining a static work posture for long time, frequent twisting motions, and repetitive same motions.

Particularly, those who are engaged in, for example, health and hygiene business and traffic and transportation business regarding which automation and mechanization of work at their workplaces are difficult are forced to do heavy work which gives large physical load to their lower back parts, their risk of developing the lower back pain is extremely high. Regarding verification of loads on an endoskeleton system which is a damaged site of this lower back pain, invasive experiments should be avoided from an ethical point of view because the target is a human body; however, it is very difficult to perform the measurement non-invasively.

Conventionally, as a method for preventing the lower back pain caused by heavy muscular work, there has been proposed a lower back pain preventing apparatus that: calculates a burden value of a lower back part (a pressure force which occurs at intervertebral disks of lumbar spines) on the basis of a trunk angle of a human body and a foot sole load; sets a burden risk value relating to the lower back part (a permissible value of the intervertebral disk pressure force), which leads to the possibility of a failure occurrence at the lower back part on the basis of the person's basic information; and sends out the risk of the work load as a warning in real time on the basis of the burden value and the burden risk value (see PTL 1).

Moreover, there is proposed a load calculation apparatus that calculates load imposed on each of a plurality of the lumbar spines on the basis of the position and orientation of each of the plurality of the lumbar spines of a user, which are obtained by calculating a curvature of the user's skin on their lower back part and its inclination angle in their upper body (see PTL 2).

Furthermore, there is proposed an alarm system capable of generating a warning to the user who takes a posture which may possibly cause the lower back pain on the basis of a height difference between the user's lower back and their body part, which causes the height difference as a result of the user taking a forward-bending posture (see PTL 3).

Additionally, there is proposed wearable devices which protect against musculoskeletal injuries and enhance performance and can be worn for extended periods of time to improve performance or reduce risk of injury, including sensors to detect various activities, motions, and postures of the wearer, and various active and semi-active control approaches which leverage sensor information to provide tailored assistance to individual users (see PTL 4).

CITATION LIST

PATENT LITERATURE

PTL 1: Japanese Patent Application Laid-Open (Kokai) Publication No. 2012-183291
PTL 2: Japanese Patent Application Laid-Open (Kokai) Publication No. 2015-134149
PTL 3: Japanese Patent Application Laid-Open (Kokai) Publication No. 2017-136313
PTL 4: WO 2019/161232 A1

SUMMARY OF THE DISCLOSURE

PROBLEMS TO BE SOLVED BY THE DISCLOSURE

Meanwhile, the heavy object lifting motion such as the heavy muscular work causes a lower back part moment equivalent to an increased weight of a heavy object at the worker's lower back part, so that the load is imposed on a musculoskeletal system of the lower back part and there is a risk of suffering the lower back pain symptom, disk herniation, etc. Therefore, an intervertebral disk pressure force which is a physical load on the intervertebral disks has been used as a load evaluation index.

However, direct measurement of the intervertebral disk pressure force to evaluate the load is not generally adopted because it requires invasiveness; and a method of estimating the intervertebral disk pressure force from, for example, the worker's work posture, a heavy object, and muscular strength to be exhibited is used. The aforementioned PTL 1 to PTL 3 also propose the method of preventing the lower back pain caused by the heavy muscular work by using various non-invasive measurement methods.

Meanwhile, PTL 1 is designed to warn the risk of the work load in real time on the basis of the intervertebral disk pressure force and its permissible value with regard to the load imposed on the lower back part when the worker performs the work; however, the degree of the burden on the lower back part with respect to the heavy object which is a work object is just instantly judged along with the trunk angle.

Moreover, PTL 2 is designed to measure loads imposed respectively on the plurality of the lumbar spines and gives different assisting forces corresponding to the respective loads to the user and to give a warning if the load which exceeds a predetermined threshold value continues for a specified amount of time; however, since the threshold value is just a predetermined value, it is difficult to forecast the possibility of chronic lower back pain which may occur for a medium-to-long period of time.

Furthermore, regarding PTL 3, when the user intends to lift an object, the possibility of the lower back pain is just judged on the basis of the height difference of the lower back which instantly occurs; and, therefore, it is difficult to estimate the risk of developing the lower back pain as caused by chronic burdens on the lower back part.

In order to indicate effectiveness of the prevention of the lower back pain, it is important to verify reduction of the loads on the endoskeleton system such as the intervertebral disks which are the damaged sites of the lower back pain; however, not only instant loads, but also repetitive loads and continuous loads are considered to be factors of the lower back pain, so that there has been a problem of serious difficulty in accurately evaluating such repetitive loads and continuous loads by using the evaluation index based only on the intervertebral disk pressure force by means of mechanics methodology.

The present invention was devised in consideration of the above-described circumstances and aims at proposing a lower back part load evaluation apparatus and a lower back part load evaluation method which use a wearable motion assistance device capable of estimating the wearer's risk of developing the lower back pain with very high accuracy.

MEANS TO SOLVE THE PROBLEMS

In order to solve the above-described problems, there is provided according to the present invention a lower back part load evaluation apparatus using a wearable motion assistance device worn by securing either a wearer's both femoral parts or the wearer's abdominal part and shoulder parts, or both of them, to assist motions of a lower back part of the wearer, wherein the lower back part load evaluation apparatus includes: a trunk angle measuring unit that continuously measures an inclination angle of the lower back part of the wearer wearing wearable motion assistance device as a trunk angle of the wearer; an intervertebral disk pressure force estimation unit that continuously estimates an intervertebral disk pressure force applied to the wearer's intervertebral disks in a vertebral-bodies-connecting direction on the basis of a weight of an object held by the wearer and a body height and body weight of the wearer which are externally input, and the trunk angle measured by the trunk angle measuring unit; a section elapsed time measuring unit that divides the intervertebral disk pressure force estimated by the intervertebral disk pressure force estimation unit into sections according to a load level and, at the same time, measures elapsed time for each of the sections in chronological order; and a lower back pain occurrence risk forecasting unit that forecasts the wearer's risk of developing a lower back pain symptom on the basis of a measurement history of the section elapsed time measuring unit during work time of heavy muscular work to handle the object.

Accordingly, the lower back part load evaluation apparatus using the wearable motion assistance device is designed to forecast the wearer's risk of developing the lower back pain symptom during the work time of the heavy muscular work to handle the object, while continuously estimating the wearer's intervertebral disk pressure force on the basis of the weight of the object and the wearer's body height and body weight, and the trunk angle of the wearer, and measuring the elapsed time in chronological order with respect to each section of the intervertebral disk pressure force according to the load level. As a result, it is possible to acquire a forecast index of the risk of developing the lower back pain symptom with respect to not only the instant loads, but also the repetitive loads and the continuous loads imposed on the wearer's lower back part.

Moreover, according to the present invention, the lower back part load evaluation apparatus using the wearable motion assistance device further includes a hip joint angle measuring unit that is provided in the wearable motion assistance device and continuously measures motion angles upon bending and stretching of right and left hip joints of the wearer, respectively, as right and left hip joint angles of the wearer, wherein the intervertebral disk pressure force estimation unit continuously estimates the intervertebral disk pressure force applied to the wearer's intervertebral disks in the vertebral-bodies-connecting direction on the basis of a combination of the trunk angle of the wearer and the right and left hip joint angles of the wearer.

As a result, the lower back part load evaluation apparatus using the wearable motion assistance device can enhance the accuracy to estimate the wearer's intervertebral disk pressure force by combining the wearer's right and left hip joint angles in addition to the wearer's trunk angle.

Furthermore, according to the present invention, the lower back pain occurrence risk forecasting unit determines, on the basis of an estimation history of the intervertebral disk pressure force estimation unit, that the wearer's risk of developing the lower back pain symptom is high when a maximum value of the intervertebral disk pressure force during the work time is close to a specified threshold limit value.

As a result, the lower back part load evaluation apparatus using the wearable motion assistance device can measure the intervertebral disk pressure force in real time during the work time and determine that there is the risk of developing the lower back pain symptom when its maximum value instantly reaches close to the threshold limit value.

Furthermore, according to the present invention, the lower back pain occurrence risk forecasting unit determines, on the basis of a measurement history of the trunk angle measuring unit, that the wearer's risk of developing the lower back pain symptom is high when the trunk angle of the wearer continues to be within a specified range to take a half-crouching posture during the work time for a specified amount of time.

As a result, the lower back part load evaluation apparatus using the wearable motion assistance device can determine that the risk of developing the lower back pain symptom is high when the wearer continues to take the half-crouching posture for a specified amount of time.

Furthermore, according to the present invention, the lower back pain occurrence risk forecasting unit calculates an average value of load imposed on the lower back part of the wearer during the work time on the basis of each measurement history of the intervertebral disk pressure force estimation unit and the section elapsed time measuring unit during the work time and determines that the wearer's risk of developing the lower back pain symptom is high when the average value becomes equal to or larger than a specified threshold value.

As a result, the lower back part load evaluation apparatus using the wearable motion assistance device can determine that the risk of developing the lower back pain symptom is high when an average of the load imposed on the wearer's lower back part during the work time becomes equal to or larger than the specified threshold value even if the load is not instantly high and temporal length of the half-crouching posture is relatively short.

Furthermore, according to the present invention, the lower back pain occurrence risk forecasting unit forecasts the wearer's risk of developing the lower back pain symptom according to a relation between a maximum value of the trunk angle of the wearer and a weight of the object during the work time on the basis of the measurement history of the trunk angle measuring unit.

As a result, the lower back part load evaluation apparatus using the wearable motion assistance device can determine that the risk of developing the lower back pain symptom is high if the weight of the object is heavier than its permissible range relative to the wearer's half-crouching posture during the work time.

Furthermore, according to the present invention, the lower back pain occurrence risk forecasting unit determines, on the basis of the measurement history of the section elapsed time measuring unit during the work time, that the wearer's risk of developing the lower back pain symptom is high when accumulated time in which the load level of the intervertebral disk pressure force during the work time belongs to a section equal to or higher than a specified level is equal to or longer than a specified period of time.

As a result, the lower back part load evaluation apparatus using the wearable motion assistance device can determine that the risk of developing the lower back pain symptom is high when the accumulated time in which the load level of the intervertebral disk pressure force during the work time belongs to the section equal to or higher than the specified level is equal to or longer than a specified period of time even if the state where the load level of the intervertebral disk pressure force during the work time is not extremely high continues.

Furthermore, according to the present invention, the lower back part load evaluation apparatus using the wearable motion assistance device further includes: a workload analysis unit that analyzes a work load for reducing load on the lower back part and an endoskeleton system of the wearer relating to the heavy muscular work on the basis of a forecasted result of the lower back pain occurrence risk forecasting unit; and an optimization control data generation unit that generates control data in order to optimize motion assistance control performance by the wearable motion assistance device on the basis of an analysis result of the workload analysis unit.

As a result, the lower back part load evaluation apparatus using the wearable motion assistance device can reduce the work load on the wearer's lower back part and the endoskeleton system relating to the heavy muscular work by optimizing the motion assistance control performance by the wearable motion assistance device.

Furthermore, according to the present invention, a lower back part load evaluation method using a wearable motion assistance device worn by securing either a wearer's both femoral parts or the wearer's abdominal part and shoulder parts, or both of them, to assist motions of a lower back part of the wearer includes: a first step of continuously measuring an inclination angle of the lower back part of the wearer wearing the wearable motion assistance device as a trunk angle of the wearer; a second step of continuously estimating an intervertebral disk pressure force applied to the wearer's intervertebral disks in a vertebral-bodies-connecting direction on the basis of a weight of an object held by the wearer and a body height and body weight of the wearer which are externally input, and the trunk angle measured in the first step; a third step of dividing the intervertebral disk pressure force estimated in the second step into sections according to a load level and, at the same time, measuring elapsed time for each of the sections in chronological order; and a fourth step of forecasting the wearer's risk of developing a lower back pain symptom on the basis of a measurement history in the third step during work time of heavy muscular work to handle the object.

Accordingly, the lower back part load evaluation method using the wearable motion assistance device is designed to forecast the wearer's risk of developing the lower back pain symptom during the work time of the heavy muscular work to handle the object, while continuously estimating the wearer's intervertebral disk pressure force on the basis of the weight of the object and the wearer's body height and the body weight, and the trunk angle of the wearer, and measuring the elapsed time in chronological order with respect to each section of the intervertebral disk pressure force according to the load level. As a result, it is possible to acquire a forecast index of the risk of developing the lower back pain symptom with respect to not only the instant loads, but also the repetitive loads and the continuous loads imposed on the wearer's lower back part.

ADVANTAGEOUS EFFECTS

According to the present disclosure, it is capable of implementing the lower back part load evaluation apparatus and the lower back part load evaluation method which use the wearable motion assistance device capable of estimating the wearer's risk of developing the lower back pain with very high accuracy.

BRIEF DESCRIPTION OF DRAWINGS

Fig. 1 is a perspective view illustrating an appearance configuration of a wearable motion assistance device according to an embodiment of the present invention;
Fig. 2 is a perspective view illustrating a major configuration of the wearable motion assistance device according to the embodiment of the present invention;
Fig. 3 is a perspective view illustrating, from a back side, a state of a wearer wearing the wearable motion assistance device;
Fig. 4 is a side view illustrating a motion status of the wearer wearing the wearable motion assistance device;
Fig. 5 is a side view illustrating a movable range of the wearable motion assistance device;
Fig. 6 is a block diagram illustrating a control system for the wearable motion assistance device;
Fig. 7 is a schematic diagram for explaining percentages of the range of motion for bending and stretching with respect to the respective the lumbar spines ;
Fig. 8 is a schematic diagram for explaining a dynamic model used for an intervertebral disk pressure force estimation theory;
Fig. 9 is a block diagram illustrating the configuration of a lower back part load evaluation apparatus;
Fig. 10 is a graph and chart for explaining a load level indicating a plurality of divided stages of an intervertebral disk pressure force; and
Fig. 11 is a graph indicating the relation between the wearer's work posture and the weight of an object held.

DESCRIPTION OF EMBODIMENTS

An embodiment of the present invention will be described below in detail with reference to the drawings.

(1) Configuration of Wearable Motion Assistance Device According to This Embodiment

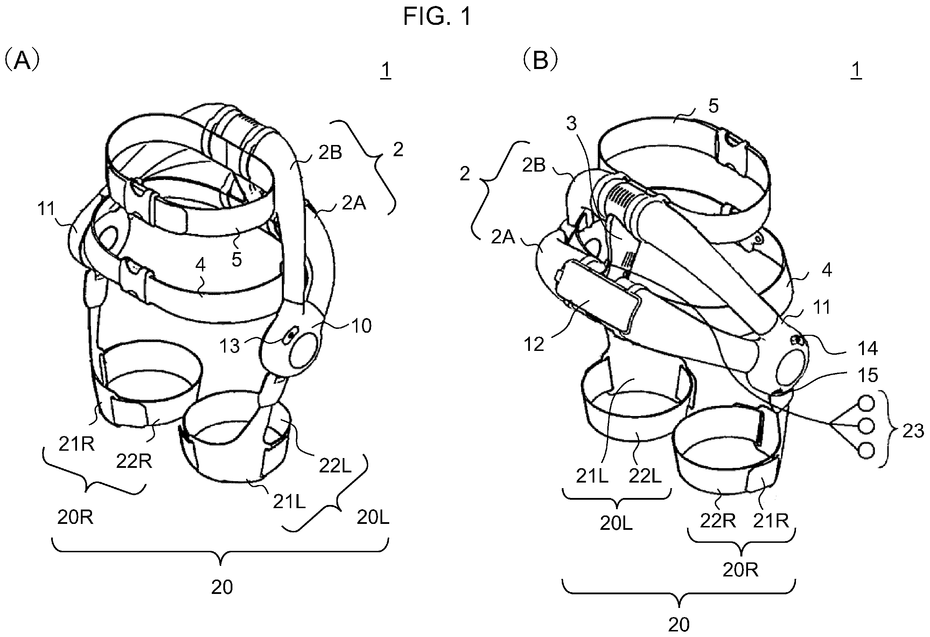

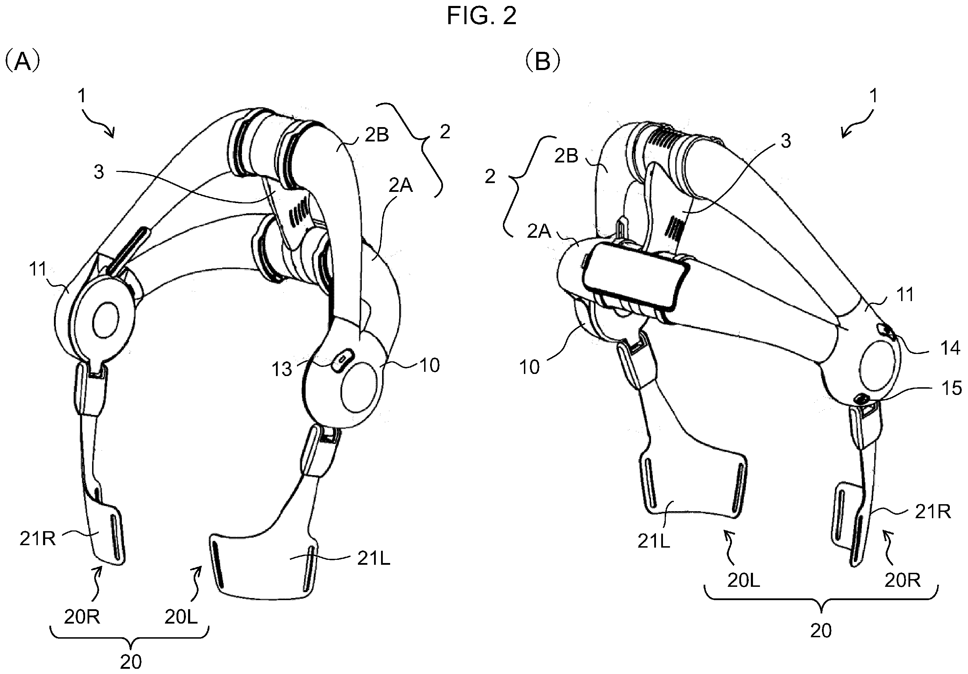

Fig. 1(A) and Fig. 1(B) illustrate a wearable motion assistance device 1 according to this embodiment. Moreover, Fig. 2(A) and Fig. 2(B) illustrate a major configuration of the wearable motion assistance device 1 in Fig. 1, excluding thigh cuffs, a belt, etc. The wearable motion assistance device 1 is a device for assisting a wearer's work and motions and operates to detect a biosignal(s) (surface EMB), which occurs when generating muscular strength according to signals from a brain, and motion angles of the wearer's hip joints and give driving power from a drive mechanism in accordance with this detected signal.

When the wearer wearing the wearable motion assistance device 1 performs a carrying motion to lift a relatively heavy object with their own will, a driving torque in accordance with a biosignal of a latissimus dorsi muscle or a gluteus maximus muscle (gluteus maximus), which then occurs, and the wearer's hip joint motion angles is given as an assisting force from the wearable motion assistance device. Therefore, the wearer can lift and carry the object with combined power of their own muscular strength and the driving torque from the drive mechanism (actuator).

Moreover, the wearable motion assistance device 1 can also assist, for example, the wearer's ascending/descending work to go up and down stairs while carrying luggage, other than the carrying work to lift the object and walk.

In the wearable motion assistance device 1, a lower-back frame 2 is mounted on a back side of the wearer's lower back across the right and left sides. The lower back part frame 2 is, for example, a hollow member made of CFRP (carbon-fiber-reinforced plastic) and has a rounded shape in accordance with the shapes of a back side and both lateral sides of a human body's lower back part.

The lower back part frame 2 is configured so that a first lower back part frame 2A which is mounted across the right and left sides and a second lower back part frame 2B which is mounted across the right and left sides above the first lower back part frame 2A are connected to each other via a support 3 on the back side of the wearer's lower back part.

The first lower back part frame 2A and the second lower back part frame 2B are mounted on the wearer's lower back part by using wearing belts 4, 5 to be extended over the wearer's abdominal side. The first lower back part frame 2A and the second lower back part frame 2B are mounted on the wearer in a forward-bent posture so that its both ends become lower than their back side in the mounted state, that is, their both ends are located at positions lower than their center parts in a lengthwise direction (a part located at the wearer's back side).

A left side frame 10 and a right side frame 11 are secured to both ends of the first lower back part frame 2A and the second lower back part frame 2B.

Moreover, a battery 12 is placed, in a freely attachable/detachable manner, in the center part of the first lower back part frame 2A and wires and the like connected to the battery 12 are inserted into, and are made to pass through, an internal space on the outside which is the opposite side of the side where the wearable motion assistance device 1 is mounted.

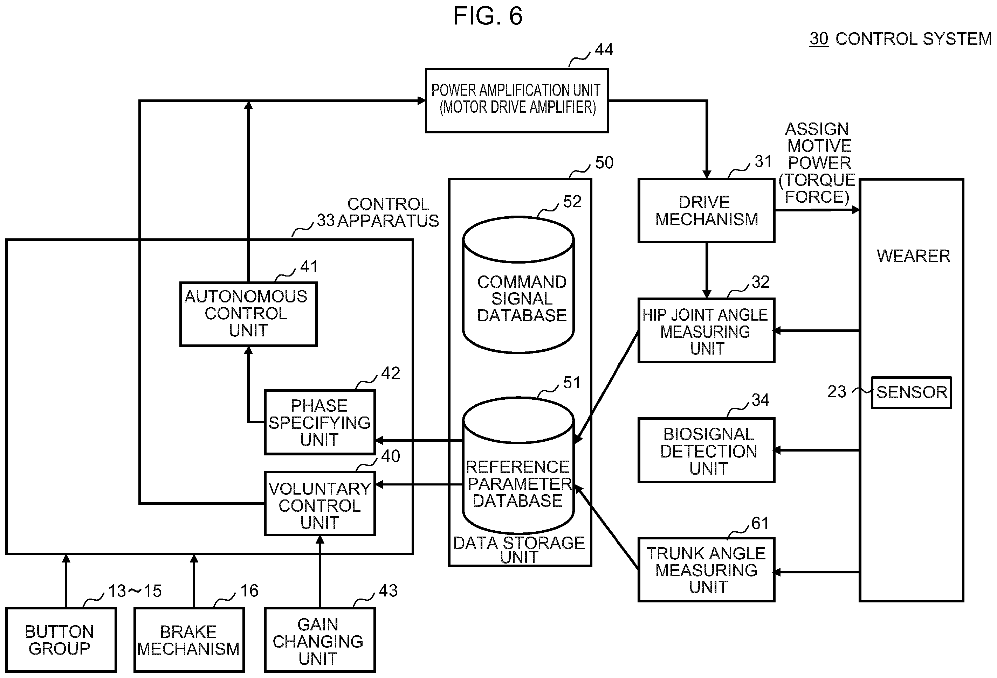

The support 3 is a member for connecting a lengthwise-direction center part of the first lower back part frame 2A and a lengthwise-direction center part of the second lower back part frame 2B in a vertical direction. The support 3 is, for example, a hollow member made of reinforced resin and wires of a sensor are inserted into, and are made to pass through, the inside of the support 3. Moreover, a control apparatus 33 (described later with reference to Fig. 6) for controlling operations of the wearable motion assistance device 1 is provided inside the support 3.

The strength of a monocoque structure is secured by using the support 3 to retain various members which constitute the first lower back part frame 2A and the second lower back part frame 2B so that they will not rotate. Moreover, the wearing belt 4 corresponding to the first lower back part frame 2A and the wearing belt 5 corresponding to the second lower back part frame 2B are respectively attached to the support 3.

The left side frame 10 is secured by joining a left end of the first lower back part frame 2A and a left end of the second lower back part frame 2B together on the left side of the wearer's hip joint. The right side frame 11 has a structure substantially bilaterally symmetrical to the structure of the left side frame 10, and is secured by joining a right end of the first lower back part frame 2A and a right end of the second lower back part frame 2B.

An actuator and a brake mechanism (both of which are not illustrated in the drawing) are built in the left side frame 10 and a minus button 13 for input to reduce a driving force of the actuator is provided. This minus button 12 lights up when the wearable motion assistance device is in a power-on state.

An actuator and a brake mechanism (both of which are not illustrated in the drawing) are built in the right side frame 11 and a plus button 14 for input to increase a driving force of the actuator and a power source button 15 for switching the power on/off of the wearable motion assistance device 1 are provided. This plus button 14 and the power source button 15 light up when the wearable motion assistance device 1 is in a power-on state.

Accordingly, the lower back part frames (the first lower back part frame 2A and the second lower back part frame 2B), the support 3, and the side frames (the left side frame 10 and the right side frame 11) are assembled to form an integrated configuration, there by realizing a monocoque structure for causing the frames themselves to receive stress.

Referring to Fig. 1(A) and Fig. 1(B), a femoral part securing unit 20 is composed of: a left thigh securing unit 20L which secures the wearer's left-side femoral part; and a right thigh securing unit 20R which secures the wearer's right-side femoral part.

The left thigh securing unit 20L is configured from a stay unit 21L connected to the actuator in the left side frame 10 and a belt unit 22L attached to the stay unit 21L and is provided in such a manner that the left thigh securing unit 20L can rotate relative to the left side frame 10 as viewed from its lateral side.

Moreover, the right thigh securing unit 20R is configured from a stay unit 21R connected to the actuator in the right side frame 11 and a belt unit 22R attached to the stay unit 21R and is provided in such a manner that the right thigh securing unit 20R can rotate relative to the right side frame 10 as viewed from its lateral side.

Incidentally, regarding the left thigh securing unit 20L and the right thigh securing unit 20R, the stay unit 21L, 21R is designed to have an optimum length with reference to an average length of a human body's femoral part and the wearer's femoral parts are secured by the belt units 22L, 22R.

The wearing belt 4 is a wearing belt wound around the abdominal side when attaching the first lower back part frame 2A to the wearer and is a main part for attaching the wearable motion assistance device 1 to the lower back part of the human body.

The wearing belt 5 is a wearing belt wound around the abdominal side when attaching the second lower back part frame 2B to the wearer. The wearing belt 5 is used to secure the wearable motion assistance device 1 to the human body at a position higher than that of the wearing belt 5 in order to efficiently transmit a reaction force, which occurs in association with movements of legs, to the wearer's abdominal part and lower back part when the wearer of the wearable motion assistance device 1 performs a motion to lift a relatively heavy object from a knee-bending state.

The battery 12 is provided on the outside of the center part of the first lower back part frame 2A on the outside of the wearable motion assistance device 1 opposite its mounted side and supplies electric power to the control apparatus 33 (Fig. 6), the actuator, a brake mechanism 16 (Fig. 6), the minus button 13, the plus button 14, and the power source button 15.

Sensors 23 are mounted on the back side of the wearer's lower back part as illustrated in Fig. 1(B) and are detection units which detect biosignals associated with muscular activities conducted when the wearer tries to raise the trunk or muscular activities conducted when the wearer tries to keep the angle of the trunk.

The sensors 23 are connected to top ends of wires extending from a hole provided in the support 3 to the outside of the support and there are three sensors 23. The sensors 23 are pasted on the back side of the wearer's lower back part, thereby detecting the biosignals which occur when the wearer moves muscles of their trunk.

The biosignals detected by the sensors 23 are input to the control apparatus 33 (Fig. 6). Incidentally, the sensors 23 may be pasted to the wearer's back by using a tape or the like or may be pasted by using a gel or the like. One of the three sensors 23 is used to measure a reference signal and the remaining two sensors are used to measure a bioelectric potential signal.

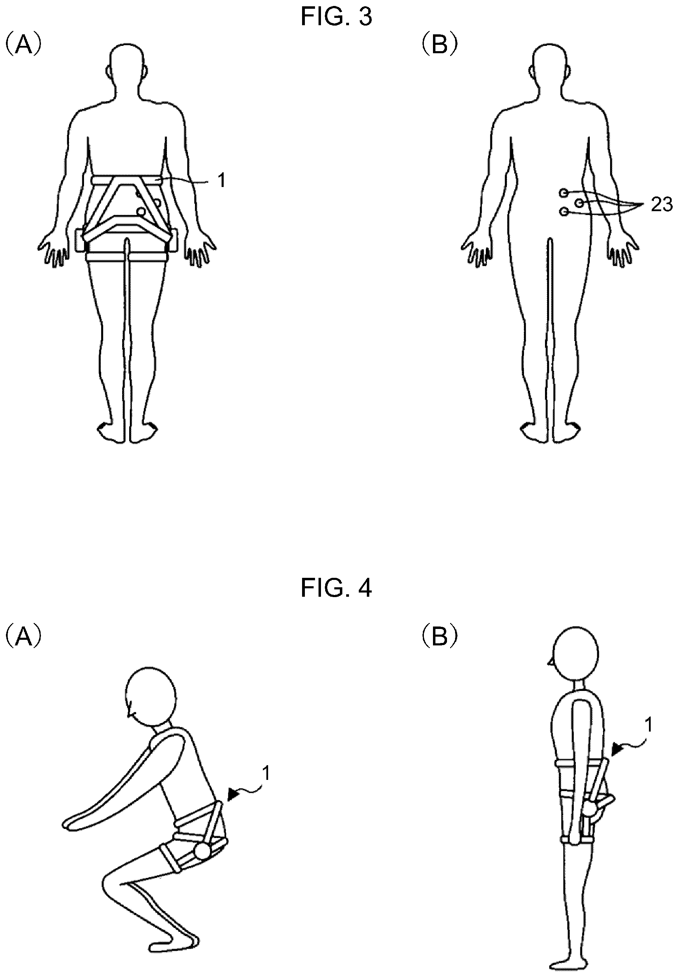

Practically, the wearable motion assistance device 1 is mounted on the wearer's lower back part from their back side as illustrated in Fig. 3(A). The wearable motion assistance device 1 is a device for generating assisting force to assist movements of the femoral parts relative to the lower back part when the wearer stands up as illustrated in Fig. 4(B) from a bent state (half-crouching posture) as illustrated in Fig. 4(A). Such movements of the wearer are, for example, a motion to stand up from the half-crouching posture, a motion to lift the object from the half-crouching posture, and a motion to assist a person moving from one place to another.

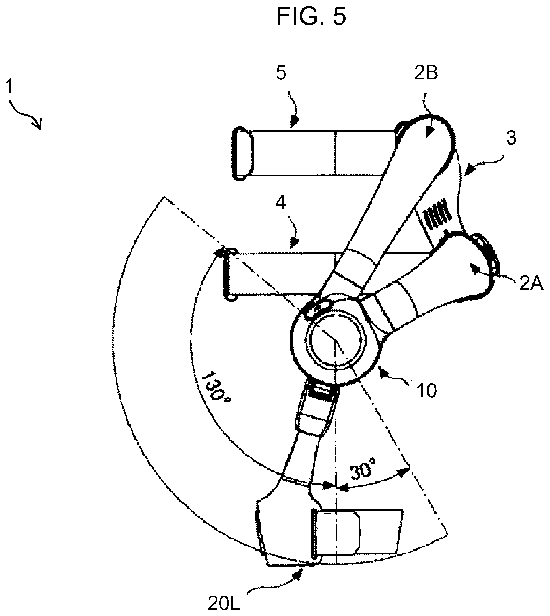

Fig. 5 is a diagram illustrating a movable range of the wearable motion assistance device 1 (left side view). The left thigh securing unit 20L can rotate 130° clockwise and rotate 30° counterclockwise around the left side frame 10 as its rotation center from a reference position illustrated in Fig. 5. As a result of such motions, the wearable motion assistance device 1 generates the assisting force to assist the movements of the femoral parts relative to the lower back part when the wearer stands up as illustrated in Fig. 4(B) from the bent-down state as illustrated in Fig. 4(A). Incidentally, the movement range of the right thigh securing unit 20R is similar to the above-described movement range.

(2) Configuration of Control System in Wearable Motion Assistance Device

Fig. 6 is a block diagram illustrating the configuration of the control system 30 for the wearable motion assistance device 1. The control system 30 for the wearable motion assistance device 1 includes, as illustrated in Fig. 6, a drive mechanism 31 which gives the assisting force to the wearer, a hip joint angle measuring unit 32 which detects the wearer's hip joint angles (physical phenomenon), and a control apparatus 33 which integrally controls the entire system.

The drive mechanism 31 drives the femoral part securing unit 20 or the lower-back frame 2 by generating a driving torque to relatively rotationally drive each of the lower-back frame 2 (the first lower back part frame 2A and the second lower back part frame 2B) which is mounted on the wearer's lower back and can be connected on both its right and left sides, and the femoral part securing unit 20 (the left thigh securing unit 20L and the right thigh securing unit 20R) which is secured to the wearer's right and left femoral parts and can be connected on the outside of the respective femoral parts.

The hip joint angle measuring unit 33 is provided in the drive mechanism 31 and detects rotation angles between the right and left sides of the lower-back frame 2 (the first lower back part frame 2A and the second lower back part frame 2B) and the femoral part securing unit 20 (the left thigh securing unit 20L and the right thigh securing unit 20R) respectively as the wearer's right and left hip joint angles. In other words, the hip joint angle measuring unit 32 continuously measures motion angles upon bending and stretching at the wearer's right and left hip joints respectively as the wearer's right and left hip joint angles.

The control apparatus 33 judges the wearer's motion and posture on the basis of the wearer's right and left hip joint angles by the hip joint angle measuring unit 32 and causes the drive mechanism 31 to generate the driving torque according to the judgment result.

Moreover, the wearable motion assistance device 1 has the biosignal detection unit 34 which detects a biosignal of the rectus femoris muscle and the gluteus maximus muscle associated with movements of the wearer's left thigh and right thigh, and a biosignal of the latissimus dorsi muscle of the wearer's back. The control apparatus 33 judges the wearer's motion and posture on the basis of the biosignals output from the biosignal detection unit 34 and the wearer's right and left hip joint angles and causes the drive mechanism 31 to generate the driving torque according to the judgment result.

When the wearer lifts the object, the drive mechanism 31 transmits the driving torque to the lower-back frame 2 (the first lower back part frame 2A and the second lower back part frame) at the same time and drives the lower-back frame towards the wearer's back side; and on the other hand side, when the wearer walks and goes up and down stairs, the drive mechanism 31 transmits the driving torque to the femoral part securing unit 20 (the left thigh securing unit 20L and the right thigh securing unit ) and drives the right and left femoral part securing units alternately in back-and-forth directions.

Incidentally, the drive mechanism 31 is composed of an actuator (drive motor). The hip joint angle measuring unit 32 is composed of an angle sensor which detects joint rotation angles. The biosignal detection unit 34 is composed of a biosignal detection sensor.

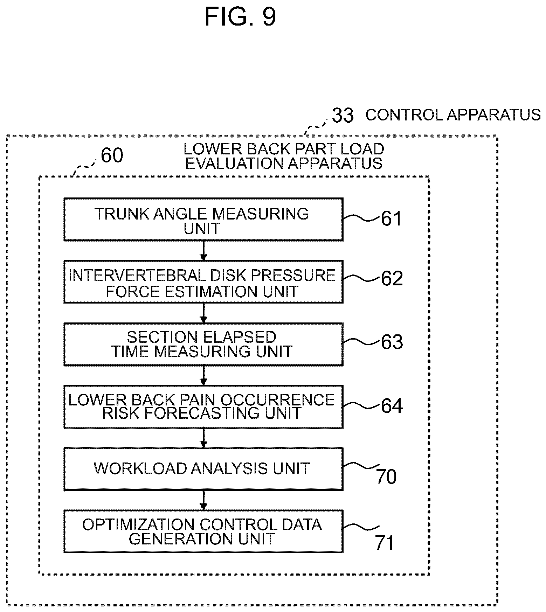

Moreover, the first lower-back frame 2A is provided with an absolute angle sensor which measures an inclination angle of the wearer's lower back part as a trunk angle measuring unit 61 (Fig. 9) described later in a lower back part load evaluation apparatus 60 (that is, a control system constituent element inside the control apparatus 33).

Moreover, a data storage unit 50 stores a reference parameter database 51 and a command signal database 52.

The control apparatus 33 is composed of, for example, a CPU (Central Processing Unit) chip having a memory and includes a voluntary control unit 40, an autonomous control unit 41, a phase specifying unit 42, and a gain changing unit 43.

The voluntary control unit 40 supplies a command signal according to the detected signal of the biosignal detection unit 34 to a power amplification unit 44. The voluntary control unit 40 generates a command signal by applying a specified command function f(t) or gain G to the biosignal detection unit 34. This gain G may be a preset value or function and can be adjusted via the gain changing unit 43.

Moreover, when it is predicted that the wearer's skin will become wet with sweat and if the biosignal from the biosignal detection unit 34 cannot be input, it is also possible to select a method of controlling the driving torque of each actuator (drive motor) on the basis of each piece of data (joint angle data detected by the angle sensor) detected by the hip joint angle measuring unit 32.

The hip joint angle detected by the hip joint angle measuring unit 32 is input to the reference parameter database 51. The phase specifying unit 42 specifies a phase of the wearer's motion by comparing the joint angle detected by the hip joint angle measuring unit 32 with a joint angle which is a reference parameter stored in the reference parameter database 51.

Then, when receiving control data of the phase specified by the phase specifying unit 42, the autonomous control unit 41 generates a command signal according to the control data of this phase and supplies the command signal for causing the drive mechanism 31 to generate motive power to the power amplification unit 44.

The data storage unit 50 has a memory for storing each piece of data and is provided with, for example, a database storage area in which phase-based control data which is set with respect to each motion pattern (task) such as a standing-up motion, a walking motion, and a sitting-down motion is stored in advance, and a control program storage area in which control programs for controlling the respective actuators are stored. The reference parameter database 51 and the command signal database 52 are stored in the database storage area.

Moreover, a button group of the minus button 13, the plus button 14, and the power source button 15 and the brake mechanism 16 which generates a braking force for rotation motions of the actuators are connected, as constituent elements, to the control apparatus 33.

When the power source button 15 is pressed, the control apparatus 33 sets the power-on state, causes the minus button 13, the plus button 14, and the power source button 15 to light up, and sets the brake mechanism to an on state; and when the femoral part securing unit 20 is moved, the control apparatus 33 generates a resistance force.

Then, when the biosignals detected by the sensors 23 are input, the control apparatus 33 causes the actuator to generate the driving force according to the drive level of the actuator which is input by the minus button 13 or the plus button 14. The minus button 13 or the plus button 14 is a button for controlling a torque tuner of the control apparatus 33 and the torque tuner can set five steps of the assisting force.

For example, when the torque tuner is set to "1," the assistance of 3 Nm at the maximum can be received with an upper limit of 20% of the assist torque; and when the torque tuner is set to "5," the assistance of 15 Nm can be received with an upper limit of 100% of the assist torque. Moreover, a torque limiter for setting the upper limit of the torque may be also provided.

When this happens, the control apparatus 33 controls the driving force according to the signal level of the biosignals which are input from the sensors. This apparatus is equipped with a Cybernic Voluntary Control (CVC) mode in order to perform the assistance.

In the CVC mode, the control system 30 inside this apparatus decides the assist torque in the following sequential order of (i) to (iii) as described below. (i) A reference assist torque is calculated from the bioelectric potential signal of the lower back part. (ii) The reference assist torque is amplified based on the value of the torque tuner. (iii) The assist torque is adjusted according to the posture and the motion content.

Moreover, upon the rotation motion of the actuator, the control apparatus 33 detects the wearer's reaction force which is input from the femoral part securing unit 20, the first lower back part frame 2A, and the second lower back part frame 2B; and if the reaction force becomes equal to or lower than a specified level, the control apparatus 33 enhances the resistance force of the brake mechanism 16 and stops the operation of the actuator. This is because the state where the reaction force becomes equal to or lower than the specified level is, for example, a state where the wearing belt 4, 5 of the femoral part securing unit 20 comes off, so that it is necessary to stop the operation in order to secure safety.

If the wearable motion assistance device 1 according to the embodiment is employed, the assisting force to assist the movements of the femoral parts relative to the lower back part can be generated when the wearer stands up as illustrated in Fig. 4(B) from the bent-down state as illustrated in Fig. 4(A).

Such an assisting force is generated as the actuator is driven on the basis of the biosignals detected by the sensors 23 in association with the muscular activities conducted when the wearer tries to raise their trunk or the muscular activities conducted when the wearer tries to maintain the angle of the trunk.

Therefore, it is possible to provide the wearable motion assistance device which is highly convenient and is capable of providing the required assisting force in a required direction in accordance with the wearer's will. Moreover, it is possible to provide the wearable motion assistance device 1 which is capable of suppressing the motive power (muscular strength), which should be generated by the wearer by themselves, and is capable of suppressing the situation where the convenience for the wearer may be impaired.

Incidentally, if the control apparatus 33 determines that signal levels of the biosignals of the wearer's right and left thighs are not equal on both the right and left sides, it is judged that the wearer is walking; and if the control apparatus 33 determines that the signal levels of the biosignals of the wearer's right and left thighs are equal on both the right and left sides, it is judged that the wearer is in a stopped state.

Moreover, if the control apparatus 33 determines, in the state where it is judged that the wearer is in the stopped state, that both the right and left hip joint angles are larger than a certain specified angle, it is judged that the wearer is walking; and, on the other hand, if the control apparatus 33 determines that both the right and left hip joint angles are equal to or smaller than a certain specified angle, it is judged that the wearer is in a posture with their upper body leaning forward and staying low.

(3) Lower Back Pain Factors Based on Human Endoskeleton Structure

A spinal column in the human skeleton serves important roles such as a support action to support the trunk and a protection action to protect the spinal cord. The spinal column is linear and bilaterally symmetrical on its frontal planes and is bent in an S-shape (physiological bowing) on its sagittal plane.

The spinal column is formed of 33 vertebrae and the vertebrae are called as follows from their top downwards: seven vertebrae are cervical spines (cervical vertebrae: C1 to C7); 12 vertebrae are thoracic spines (thoracic vertebrae: T1 to T12); five vertebrae are lumbar spines (lumbar vertebrae: L1 toL5); five vertebrae are sacral vertebrae (sacrum: S1 to S5); and four vertebrae are caudal vertebrae (coccyx).

The respective vertebrae are connected to each other by their intervertebral disks on the front side and by a vertebral arch and are aligned in a row. An intervertebral disk existing between the vertebrae has a nucleus pulposus in its center and the nucleus pulposus is surrounded by a fibrous ring, and front and back ligaments serve as a buffer role and make the intervertebral disk stays between the vertebral bodies.

The vertebrae are formed of circular vertebral bodies at the front and an arch-shaped vertebral arch at the back and the vertebral arch includes superior and inferior articular processes, spinous processes, and transverse processes. The vertebral bodies and the intervertebral disks are called a front spinal column and serve a role to support the spinal columns. Moreover, the vertebral arch and condyloid processes are called a back spinal column and serve a motor control function.

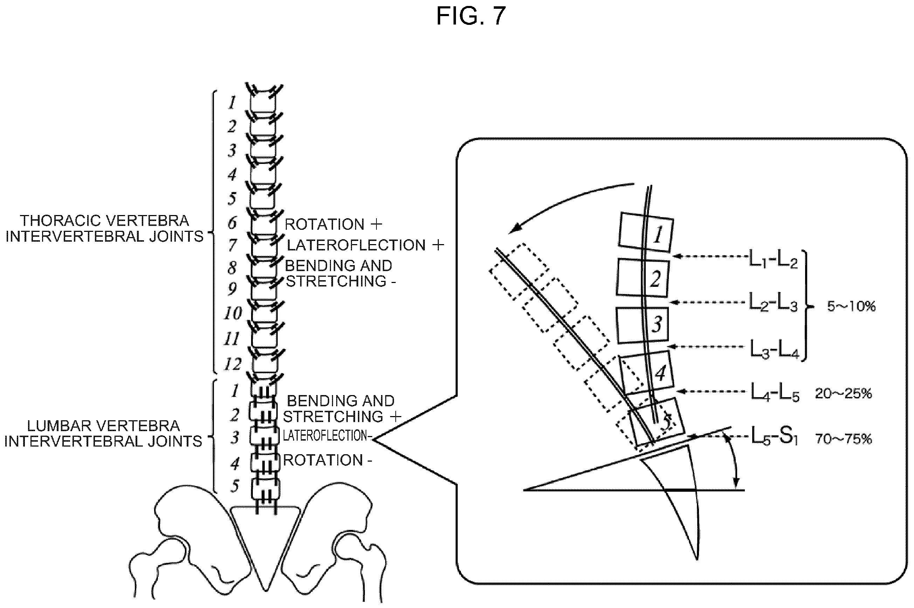

What is important regarding the motions of the spinal column is the connection between the intervertebral joints and the vertebral bodies and Fig. 7 illustrates differences in intervertebral joint facets between the thoracic spines and the lumbar spines. Since the intervertebral joints of the cervical spines have articular facets which are close to flat planes, those other than superior cervical spines perform bending, stretching, and lateroflection. Turning motions are performed at C1, C2. The intervertebral joints of the thoracic spines take externally open slanting positions and a thorax is associated with them at the same time, so that they can turn and perform lateroflection, but cannot perform bending or stretching.

Regarding the lumbar spines, their articular facets are vertical, so that they cannot turn or perform lateroflection, but can only perform the bending and stretching. Referring to Fig. 7, it is said that the bending and stretching motion of the lumbar spines is performed at the percentage of 5% to 10% between the first and second lumbar spines (L1-L2), the second and third lumbar spines (L2-L3), and the third and fourth lumbar spines (L3-L4), at 20% between the fourth and fifth lumbar spines (L4-L5), and at 70% to 75% between the fifth lumbar spine and the sacral vertebra (L5-S1).

The lower back pain includes physical factors and psychological factors and most of the physical factors are caused by damage to the muscles, ligaments, bones, and intervertebral disks. A representative example of the intervertebral disk damage is a disk herniation (slipped disk). The herniation indicates a state where an organ or the like inside a body is slipped out of its original site and the disk herniation is the state where part of the intervertebral disk protrudes beyond a normal intervertebral space.

The intervertebral disks existing at L5-S1, among the lumbar spines exist in a transition part between movable spines and unmovable spines, so that a heavy load is imposed on them when bending the trunk and the intervertebral disks tend to easily suffer damage. Therefore, 95% of the disk herniation occur at the intervertebral disks of L4-L5 and L5-S1. Sensory fibers are distributed around the intervertebral disks; and when the wearer takes a posture which increases the internal intervertebral disk pressure, nerves of the sensory fibers are stimulated, thereby causing the pain.

(4) Intervertebral Disk Pressure Force Estimation Theory and Lower Back Part Load

An explanation will be provided below about a method for estimating, as a load evaluation index, an intervertebral disk pressure force which is a physical load on the intervertebral disks from the work posture, the object, the muscular strength to be exhibited, and so on.

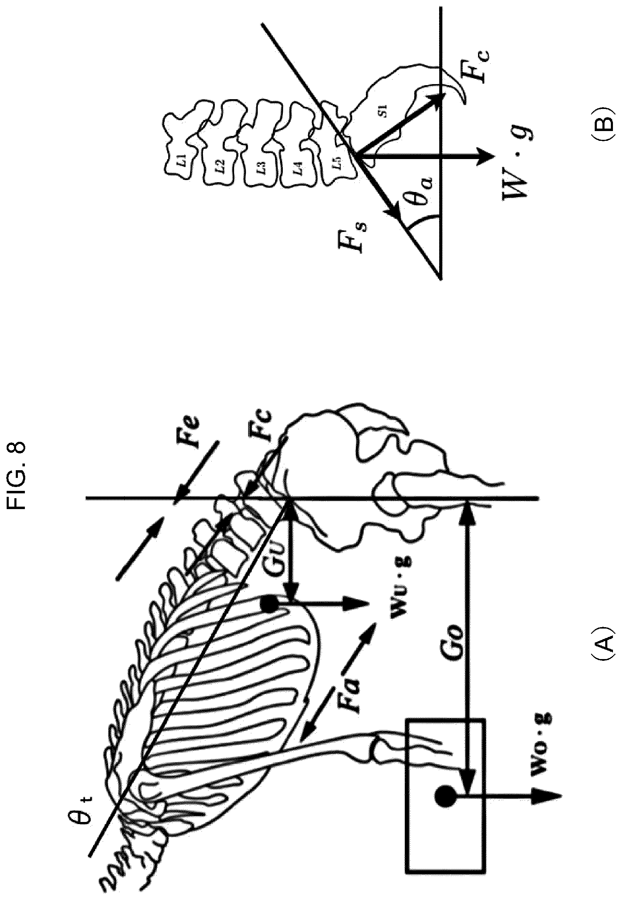

An explanation will be provided about formulas for estimating the intervertebral disk pressure force imposed on the L5-S1 intervertebral disks during two-dimensional static work with respect to a dynamic model illustrated in Fig. 8(A). The weight $W_u$ [kg] of an upper body can be approximated as expressed in the following Expression (1), where $W_b$[kg] represents the wearer's body weight.

[Math. 1]

$$W_u = 0.475 W_b \qquad\qquad \cdots\cdots\ (1)$$

A horizontal distance $G_u$[m] between the L5-S1 intervertebral disk(s) to the center of gravity of the upper body can be approximated as expressed in the following expression (2), where $L_b$ [m] represents the wearer's body height and $\theta_t$ [deg] represents the trunk angle (an angle formed between a line extending between shoulders and a greater trochanter and a vertical line).

[Math. 2]

$$G_u = 0.17 L_b \cdot sin\theta_t \qquad\qquad \cdots\cdots\ (2)$$

Similarly, a horizontal distance $G_o$ [m] to the center of gravity of the object is found according to the following Expression (3), where $L_s$ [m] represents the distance between the wearer's intervertebral disks and their shoulders.

[Math. 3]

$$G_o = L_s \cdot sin\theta_t \qquad\qquad \cdots\cdots\ (3)$$

A lower back part moment $M_l$ [Nm] around the L5-S1 intervertebral disks, which is caused by the wearer's body weight and the weight of the object is found according to the following Expression (4). However, g [Nm/s²] represents gravitational acceleration and $W_o$ [kg] represents the weight of the held object.

[Math. 4]

$$M_l = W_u \cdot g \cdot G_u + W_o \cdot g \cdot G_o \qquad\qquad \cdots\cdots\ (4)$$

Muscles which stabilize the lumbar spines include an erector spinae muscle group and an abdominal muscle group such as rectus abdominis muscles, internal and external oblique muscles of the abdomen, and transverse abdominal muscles. An abdominal cavity is a body cavity which is filled with liquids and things close to semi-fluids and can be sealed. An increase of an intraabdominal pressure occurs due to shrinkage of the abdominal muscle group and a diaphragm. If the pressure is applied to the space of the abdominal cavity, a force to retain it is generated and the force applied to the spinal columns is distributed, thereby decreasing the pressure force. However, if the body is strongly bent forward or the trunk of the body is bent in a half-crouching posture, an abdominal wall sags and the abdominal pressure thereby decreases.

The force $F_a$ [N] caused by the intraabdominal pressure can be expressed by the following Expression (5) according to Fisher's estimation formula. However, $\theta_h$ [deg] represents the joint angle of the hip joint and $A_d$ [m²] represents a diaphragm area and 0.0465 [m²] is used.

[Math. 5]

$$F_a = 0.001\{42.64 - 0.3564(180 - \theta_h)\}M_l^{11.8} \cdot 13.6 \cdot g \cdot A_d \qquad \cdots\cdots (5)$$

A moment arm $D_a$ [m] of the intraabdominal pressure is estimated as expressed in the following Expression (6) by using the joint angle $\theta_h$ of the hip joint according to the estimation formula of Morris et al.
[Math. 6]

$$D_a = 0.067 + 0.082 sin\theta_h \qquad \cdots\cdots (6)$$

The force $F_e$ [N] caused by the erector spinae muscles is found by the following Expression (7). However, $D_e$ [m] represents a moment arm of the erector spinae muscles and 0.05 [m] is used.
[Math. 7]

$$F_e = (M_l - F_a \cdot D_a)/D_e \qquad \cdots\cdots (7)$$

Since the articular facet of the L5-S1 intervertebral disks is inclined as illustrated in Fig. 8(B), the intervertebral disk pressure force and an intervertebral disk shearing force are imposed on it and proportions of these respective forces depend on the inclination angle of the relevant articular facet. The articular facet of the L5-S1 intervertebral disks is determined by a rotation angle of a pelvis; and the pelvis rotation increases as the trunk is bent further, while the pelvis rotation decrease when the hip joints and knee joints are bent in the half-crouching posture.

The inclination angle $\theta_a$ [deg] of the articular facet of the L5-S1 intervertebral disks is estimated by the following Expression (8) according to the estimation formula of Anderson et al. However, $\theta_k$ [deg] represents the joint angle of the knee joint. Incidentally, the joint angle $\theta_k$ of the knee joint can be estimated from the joint angle $\theta_h$ of the hip joint and the trunk angle $\theta_t$ which are measured.
[Math. 8]

$$\theta_a = -17.519 - 0.11863\theta_t + 0.2268\theta_k + 0.0011904\theta_t \cdot \theta_k + 0.00499\theta_t^2 -$$
$$0.000753\theta_t^2 + 40 \qquad \cdots\cdots (8)$$

The intervertebral disks are damaged by the intervertebral disk pressure force which presses the intervertebral disks vertically and an intervertebral disk pressure force $F_c$ [Nm] is found according to the following Expression (9).
[Math. 9]

$$F_c = W_u \cdot g \cdot cos\theta_a + W_o \cdot g \cdot cos\theta_a - F_a + F_e \qquad \cdots\cdots (9)$$

An intervertebral disk shearing force $F_s$ [Nm] is found according to the following Expression (10).
[Math. 10]

$$F_s = W_u \cdot g \cdot sin\theta_a + W_o \cdot g \cdot sin\theta_a \qquad \cdots\cdots (10)$$

The permissible limit of the intervertebral disk pressure force imposed on the L5-S1 intervertebral disks is determined as 3,400 [N] by NIOSH (National Institute for Occupational Safety Health); and if the intervertebral disk pressure force exceeds this value, it is said that the lower back pain will be caused.

For example, if the aforementioned Expression (9) is calculated under the condition that a person with a body height of 1.75 [m] and body weight of 65 [kg] holds an object of 30 [kg] with a posture bending their trunk at 60 [deg], approximately 3,525 [N] of the intervertebral disk pressure force will be imposed on the L5-S1 intervertebral disks. This shows that the intervertebral disk pressure force imposed on the L5-S1 intervertebral disks exceeds the permissible limit.

(5) Configuration of Lower Back Part Load Evaluation Apparatus According to This Embodiment

According to the present invention, the lower back part load evaluation apparatus 60 using the aforementioned wearable motion assistance device 1 is designed to estimate the wearer's risk of developing the lower back pain symptom with very high accuracy.

This lower back part load evaluation apparatus 60 is a control system constituent element provided in the control apparatus 33 in the wearable motion assistance device 1 illustrated in Fig. 6 explained earlier and includes, as illustrated in Fig. 9, the trunk angle measuring unit 61, an intervertebral disk pressure force estimation unit 62, a section elapsed time measuring unit 63, and a lower back pain occurrence risk forecasting unit 64. Furthermore, the lower back part load evaluation apparatus 60 also includes a workload analysis unit 70 and an optimization data generation unit 71 for optimizing the control performance for the motion assistance by the wearable motion assistance device 1 as described later.

The trunk angle measuring unit 61 continuously measures an inclination angle of the wearer's lower back part as the wearer's trunk angle by using an absolute angle sensor attached to the lower-back frame 2 (particularly, the first lower-back frame 2A).

The intervertebral disk pressure force estimation unit 62 continuously measures the intervertebral disk pressure force applied to the wearer's intervertebral disks in the vertebral-bodies-connecting direction on the basis of the weight of the object held by the wearer and the wearer's body height and body weight, which are input externally, and the trunk angle measured by the trunk angle measuring unit 61.

Furthermore, in order to enhance the accuracy to estimate the wearer's intervertebral disk pressure force, the intervertebral disk pressure force estimation unit 62 may continuously estimate the intervertebral disk pressure force applied to the wearer's intervertebral disks in the vertebral-bodies-connecting direction on the basis of a combination of the wearer's trunk angle and the wearer's right and left hip joint angles.

Incidentally, the weight of the object may be measured by mounting a floor reaction force sensor (which is not shown in the drawing) on each of the wearer's legs and measuring the weight of the object as an amount increased by holding the object. Also, the wearer's body height and body weight are input by using a specified input device (which is not shown in the drawing) attached to the wearable motion assistance device 1.

The section elapsed time measuring unit 63 divides the intervertebral disk pressure force estimated by the intervertebral disk pressure force estimation unit 62 into sections according to the load level and, at the same time, measures elapsed time with respect to each section in chronological order.

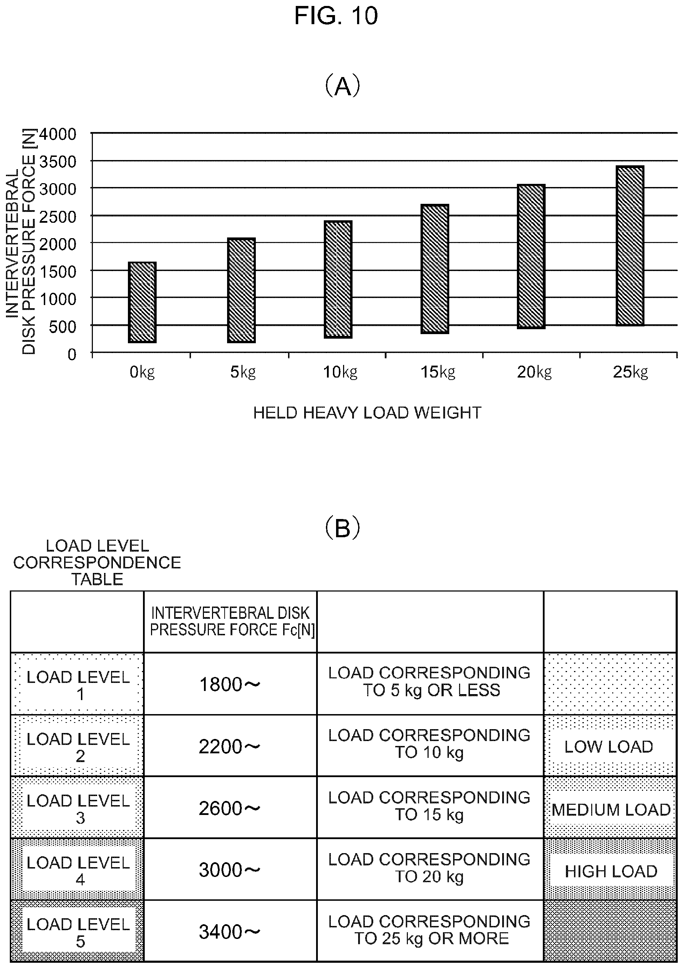

The intervertebral disk pressure force is divided into five load levels as illustrated in Fig. 10(B) on the basis of the relation between the weight of the object held and the intervertebral disk pressure as illustrated in Fig. 10(A). Specifically speaking, the intervertebral disk pressure force is divided into sections of: load level 1 when the intervertebral disk pressure force is less than 1,800 [N]; load level 2 (low load) when the intervertebral disk pressure force is 1,800 [N] or more and less than 2,200 [N]; load level 3 (medium load) when the intervertebral disk pressure force is 2,200 [N] or more and less than 2,600 [N]; load level 4 when the intervertebral disk pressure force is 2,600 [N] or more and less than 3,000 [N]; and load level 5 (high load) when the intervertebral disk pressure force is 3,000 [N] or more.

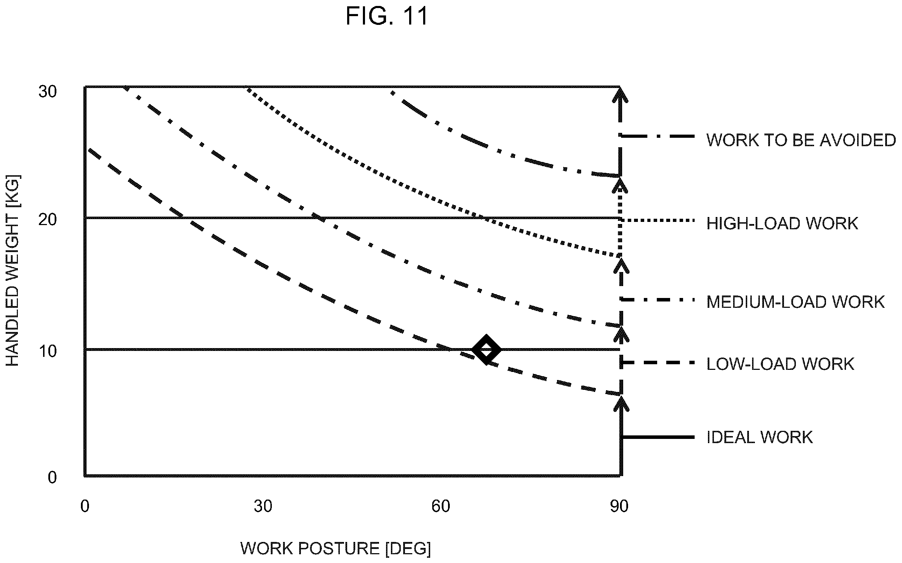

Furthermore, Fig. 11 illustrates the relation between the wearer's work posture and the weight of the held object. For example, when the weight of the object is 10 [kg], you can see that work of a low load level will be performed where the wearer's trunk angle is 70 [deg].

The lower back pain occurrence risk forecasting unit 64 forecasts the wearer's risk of developing the lower back pain symptom on the basis of the measurement history of the section elapsed time measuring unit 63 during the work time of the heavy muscular work to handle the object.

Accordingly, the lower back part load evaluation apparatus 60 using the wearable motion assistance device 1 is designed to forecast the wearer's risk of developing the lower back pain symptom during the work time of the heavy muscular work to handle the object, while continuously estimating the wearer's intervertebral disk pressure force on the basis of the weight of the object and the wearer's body height and body weight, and the trunk angle of the wearer, and measuring the elapsed time in chronological order with respect to each section of the intervertebral disk pressure force according to the load level. As a result, it is possible to acquire a forecast index of the risk of developing the lower back pain symptom with respect to not only the instant loads, but also the repetitive loads and the continuous loads imposed on the wearer's lower back part.

Moreover, with the lower back part load evaluation apparatus 60, the lower back pain occurrence risk forecasting unit 64 determines, on the basis of the estimation history of the intervertebral disk pressure force estimation unit 62, that the wearer's risk of developing the lower back pain symptom is high when a maximum value of the intervertebral disk pressure force during the work time is close to a specified threshold limit value (3,400 [N]).

As a result, the lower back part load evaluation apparatus 60 can measure the intervertebral disk pressure force in real time during the work time and determine, regardless of the load level sections, that there is the risk of developing the lower back pain symptom when its maximum value instantly reaches close to the threshold limit value.

Furthermore, with the lower back part load evaluation apparatus 60, the lower back pain occurrence risk forecasting unit 64 determines, on the basis of the measurement history of the trunk angle measuring unit 61, that the wearer's risk of developing the lower back pain symptom is high when the trunk angle of the wearer continues to be within a specified range (for example, $45 \pm 3$ [deg]) to take a half-crouching posture during the work time for a specified period of time. As a result, the lower back part load evaluation apparatus 60 can determine that the risk of developing the lower back pain symptom is high when the wearer's half-crouching posture continues for the specified period of time.

Furthermore, with the lower back part load evaluation apparatus 60, the lower back pain occurrence risk forecasting unit 64 calculates an average value of load imposed on the lower back part of the wearer during the work time on the basis of each measurement history of the intervertebral disk pressure force estimation unit 62 and the section elapsed time measuring unit 63 and determines that the wearer's risk of developing the lower back pain symptom is high when the average value becomes equal to or larger than a specified threshold value.

As a result, the lower back part load evaluation apparatus 60 can determine that the risk of developing the lower back pain symptom is high when an average of the load imposed on the wearer's lower back part during the work time becomes equal to or larger than the specified threshold value even if the load is not instantly high and temporal length of the half-crouching posture is relatively short.

Furthermore, with the lower back part load evaluation apparatus 60, the lower back pain occurrence risk forecasting unit 64 forecasts the wearer's risk of developing the lower back pain symptom according to a relation between a maximum value of the trunk angle of the wearer and a weight of the object during the work time on the basis of the measurement history of the trunk angle measuring unit 61.

As a result, the lower back part load evaluation apparatus 60 can determine that the risk of developing the lower back pain symptom is high if the weight of the object is heavier than its permissible range relative to the wearer's half-crouching posture during the work time.

Furthermore, with the lower back part load evaluation apparatus 60, the lower back pain occurrence risk forecasting unit 64 determines, on the basis of the measurement history of the section elapsed time measuring unit 63 during the work time, that the wearer's risk of developing the lower back pain symptom is high when accumulated time in which the load level of the intervertebral disk pressure force during the work time belongs to a section equal to or higher than a specified level is equal to or longer than a specified period of time.

As a result, the lower back part load evaluation apparatus 60 can determine that the risk of developing the lower back pain symptom is high when the accumulated time in which the load level of the intervertebral disk pressure force during the work time belongs to the section equal to or higher than the specified level is equal to or longer than a specified period of time even if the state where the load level of the intervertebral disk pressure force during the work time is not extremely high continues.

Furthermore, with the lower back part load evaluation apparatus 60, the workload analysis unit 70 analyzes a work load for reducing load on the wearer's lower back part and endoskeleton system relating to the heavy muscular work on the basis of a forecasted result of the lower back pain occurrence risk forecasting unit 64. Then, the optimization control data generation unit 71 generates control data in order to optimize motion assistance control performance by the wearable motion assistance device 1 on the basis of an analysis result of the workload analysis unit 70.

Examples of the motion assistance control performance by the wearable motion assistance device 1 include average output torque and maximum output torque of the drive mechanism (actuator) 31, and torque tuner settings.

As a result, the lower back part load evaluation apparatus 60 which uses the wearable motion assistance device 1 can reduce the work load on the wearer's lower back part and endoskeleton system relating to the heavy muscular work by optimizing the motion assistance control performance by the wearable motion assistance device 1.

(6) Other Embodiments

Incidentally, this embodiment has described the case where the device which is configured to be worn by securing the wearer's both femoral parts and abdominal part, and assist the motions of the wearer's lower back part is applied as the wearable motion assistance device; however, a device which is designed to be worn by securing either the wearer's both femoral parts or their abdominal part and shoulder parts, or both of them, as long as it can assist the motions of the wearer's lower back part can be envisioned. The invention is defined by the appended claims.

REFERENCE SIGNS LIST

| 1: | wearable motion assistance device |
|---|---|
| 2: | lower-back frame |
| 2A: | first lower-back frame |
| 2B: | second lower-back frame |
| 3: | support |
| 4, 5: | wearing belts |
| 10: | left side frame |
| 11: | right side frame |
| 12: | battery |
| 13: | minus button |

| 14: | plus button |
|---|---|
| 15: | power source button |
| 16: | brake mechanism |
| 20: | femoral part securing unit |
| 20L: | left thigh securing unit |
| 20R: | right thigh securing unit |
| 21L, 21R: | stay unit |
| 22L, 22R: | belt unit |
| 30: | control system |
| 31: | drive mechanism |
| 32: | hip joint angle measuring unit |
| 33: | control apparatus |
| 34: | biosignal detection unit |
| 40: | voluntary control unit |
| 41: | autonomous control unit |
| 42: | phase specifying unit |
| 43: | gain changing unit |
| 44: | power amplification unit |
| 50: | data storage unit |
| 51: | reference parameter database |
| 52: | command signal database |
| 60: | lower back part load evaluation apparatus |
| 61: | trunk angle measuring unit |
| 62: | intervertebral disk pressure force estimation unit |
| 63: | section elapsed time measuring unit |
| 64: | lower back pain occurrence risk forecasting unit |
| 70: | workload analysis unit |
| 71: | optimization control data generation unit |

**Claims**

1. A lower back part load evaluation apparatus (60) comprising a wearable motion assistance device (1) is configured to be worn by securing either a wearer's both femoral parts or the wearer's abdominal part and shoulder parts, or both of them, to assist motions of a lower back part of the wearer,
the lower back part load evaluation apparatus (60) comprising:

   a trunk angle measuring unit (61) that is provided in the wearable motion assistance device (1) and is configured to continuously measure an inclination angle of the lower back part of the wearer as a trunk angle of the wearer; **characterized by** an intervertebral disk pressure force estimation unit (62) that is configured to continuously estimate an intervertebral disk pressure force applied to the wearer's intervertebral disks in a vertebral-bodies-connecting direction on the basis of a weight of an object held by the wearer and a body height and body weight of the wearer which are externally input, and the trunk angle measured by the trunk angle measuring unit (61);
   a section elapsed time measuring unit (63) that is configured to divide the intervertebral disk pressure force estimated by the intervertebral disk pressure force estimation unit (62) into sections according to a load level and is configured to, at the same time, measure elapsed time for each of the sections in chronological order; and
   a lower back pain occurrence risk forecasting unit (64) that is configured to forecast the wearer's risk of developing a lower back pain symptom on the basis of a measurement history of the section elapsed time measuring unit (63) during work time of heavy muscular work to handle the object.

2. The lower back part load evaluation apparatus (60) comprising the wearable motion assistance device (1) according to claim 1,

   further comprising a hip joint angle measuring unit (32) that is provided in the wearable motion assistance device (1) and is configured to continuously measure motion angles upon bending and stretching of right and left hip joints of the wearer, respectively, as right and left hip joint angles of the wearer,
   wherein the intervertebral disk pressure force estimation unit (62) is configured to continuously estimate the intervertebral disk pressure force applied to the wearer's intervertebral disks in the vertebral-bodies-connecting direction on the basis of a combination of the trunk angle of the wearer and the right and left hip joint angles of the

15

wearer.

3. The lower back part load evaluation apparatus (60) comprising the wearable motion assistance device (1) according to claim 1 or 2, wherein the lower back pain occurrence risk forecasting unit (64) is configured to determine, on the basis of an estimation history of the intervertebral disk pressure force estimation unit (62), that the wearer's risk of developing the lower back pain symptom is high when a maximum value of the intervertebral disk pressure force during the work time is close to a specified threshold limit value.

4. The lower back part load evaluation apparatus (60) comprising the wearable motion assistance device (1) according to any one of claims 1 to 3, wherein the lower back pain occurrence risk forecasting unit (64) is configured to determine, on the basis of a measurement history of the trunk angle measuring unit (61), that the wearer's risk of developing the lower back pain symptom is high when the trunk angle of the wearer continues to be within a specified range to take a half-crouching posture during the work time for a specified amount of time.

5. The lower back part load evaluation apparatus (60) comprising the wearable motion assistance device (1) according to any one of claims 1 to 4, wherein the lower back pain occurrence risk forecasting unit (64) is configured to calculate an average value of load imposed on the lower back part of the wearer during the work time on the basis of each measurement history of the intervertebral disk pressure force estimation unit (62) and the section elapsed time measuring unit (63) during the work time and determine that the wearer's risk of developing the lower back pain symptom is high when the average value becomes equal to or larger than a specified threshold value.

6. The lower back part load evaluation apparatus (60) comprising the wearable motion assistance device (1) according to any one of claims 1 to 5, wherein the lower back pain occurrence risk forecasting unit (64) is configured to forecast the wearer's risk of developing the lower back pain symptom according to a relation between a maximum value of the trunk angle of the wearer and a weight of the object during the work time on the basis of the measurement history of the trunk angle measuring unit (61).

7. The lower back part load evaluation apparatus (60) comprising the wearable motion assistance device (1) according to any one of claims 1 to 6, wherein the lower back pain occurrence risk forecasting unit (64) is configured to determine, on the basis of the measurement history of the section elapsed time measuring unit (63) during the work time, that the wearer's risk of developing the lower back pain symptom is high when accumulated time in which the load level of the intervertebral disk pressure force during the work time belongs to a section equal to or higher than a specified level is equal to or longer than a specified period of time.

8. The lower back part load evaluation apparatus (60) comprising the wearable motion assistance device (1) according to any one of claims 1 to 7, further comprising: a workload analysis unit (70) that is configured to analyse a work load for reducing load on the lower back part and an endoskeleton system of the wearer relating to the heavy muscular work on the basis of a forecasted result of the lower back pain occurrence risk forecasting unit (64); and an optimization control data generation unit (71) that is configured to generate control data in order to optimize motion assistance control performance by the wearable motion assistance device (1) on the basis of an analysis result of the workload analysis unit (70).

9. The lower back part load evaluation apparatus (60) comprising the wearable motion assistance device (1) according to any one of claims 1 to 8, wherein the wearable motion assistance device (1) includes:

a drive mechanism (31) that is configured to drive femoral part securing units (20) or a lower-back frame (2) by generating a driving torque to relatively rotationally drive each of the lower-back frame (2) which is configured to be mounted on the lower back of the wearer and can be connected to its right and left sides, and the femoral part securing units (20) which are configured to be secured to right and left femoral parts of the wearer and can be connected to an outside of each of the femoral parts; a hip joint angle measuring unit (32) that is provided in the drive mechanism (31) and detects rotation angles between the lower-back frame (2) and the femoral part securing units (20) respectively as right and left hip joint angles of the wearer; and

a control unit (33) that judges a motion and posture of the wearer on the basis of the right and left hip joint angles of the wearer by the hip joint angle measuring unit (32) and causes the drive mechanism (31) to generate a driving torque according to a result of the judgment.

10. The lower back part load evaluation apparatus (60) comprising the wearable motion assistance device (1) according to claim 9,

further comprising a biosignal detection unit (34) that is configured to detect a biosignal of a rectus femoris muscle and a gluteus maximus muscle associated with movements of the wearer's left thigh and right thigh and a biosignal of a latissimus dorsi muscle of the wearer's back,

wherein the control unit (33) is configured to judge the wearer's motion and posture on the basis of the biosignals output from the biosignal detection and the right and left hip joint angles of the wearer and causes the drive mechanism (31) to generate a driving torque according to a result of the judgment.

11. A lower back part load evaluation method using a wearable motion assistance device (1) worn by securing either a wearer's both femoral parts or the wearer's abdominal part and shoulder parts, or both of them, to assist motions of a lower back part of the wearer,

the lower back part load evaluation method comprising:

a first step of continuously measuring an inclination angle of the lower back part of the wearer wearing the wearable motion assistance device (1) as a trunk angle of the wearer;

a second step of continuously estimating an intervertebral disk pressure force applied to the wearer's intervertebral disks in a vertebral-bodies-connecting direction on the basis of a weight of an object held by the wearer and a body height and body weight of the wearer which are externally input, and the trunk angle measured in the first step;

a third step of dividing the intervertebral disk pressure force estimated in the second step into sections according to a load level and, at the same time, measuring elapsed time for each of the sections in chronological order; and

a fourth step of forecasting the wearer's risk of developing a lower back pain symptom on the basis of a measurement history in the third step during work time of heavy muscular work to handle the object.

12. The lower back part load evaluation method using the wearable motion assistance device (1) according to claim 11,

wherein in the first step, motion angles upon bending and stretching of right and left hip joints of the wearer are continuously measured, respectively, as right and left hip joint angles of the wearer,

wherein in the second step, the intervertebral disk pressure force applied to the wearer's intervertebral disks in the vertebral-bodies-connecting direction is continuously estimated on the basis of a combination of the trunk angle of the wearer and the right and left hip joint angles of the wearer.

13. The lower back part load evaluation method using the wearable motion assistance device (1) according to claim 11 or 12,

wherein in the fourth step, it is determined, on the basis of an estimation history of the third step, that the wearer's risk of developing the lower back pain symptom is high when a maximum value of the intervertebral disk pressure force during the work time is close to a specified threshold limit value.

14. The lower back part load evaluation method using the wearable motion assistance device (1) according to any one of claims 11 to 13,

wherein in the fourth step, it is determined, on the basis of a measurement history of the first step, that the wearer's risk of developing the lower back pain symptom is high when the trunk angle of the wearer continues to be within a specified range to take a half-crouching posture during the work time for a specified amount of time.

15. The lower back part load evaluation method using the wearable motion assistance device (1) according to any one of claims 11 to 14,

wherein in the fourth step, an average value of load imposed on the lower back part of the wearer during the work time is calculated on the basis of each measurement history of the second step and the third step and it is determined that the wearer's risk of developing the lower back pain symptom is high when the average value becomes equal to or larger than a specified threshold value.

**16.** The lower back part load evaluation method using the wearable motion assistance device (1) according to any one of claims 11 to 15,
wherein in the fourth step, the wearer's risk of developing the lower back pain symptom according to a relation between a maximum value of the trunk angle of the wearer and a weight of the object during the work time is forecasted on the basis of the measurement history of the first step.

**17.** The lower back part load evaluation method using the wearable motion assistance device (1) according to any one of claims 11 to 16,
wherein in the fourth step, it is determined, on the basis of the measurement history of the third step during the work time, that the wearer's risk of developing the lower back pain symptom is high when accumulated time in which the load level of the intervertebral disk pressure force during the work time belongs to a section equal to or higher than a specified level is equal to or longer than a specified period of time.

**18.** The lower back part load evaluation method using the wearable motion assistance device (1) according to any one of claims 11 to 17, further comprising:

a fifth step of analyzing a work load for reducing load on the lower back part and an endoskeleton system of the wearer relating to the heavy muscular work on the basis of a forecasted result of the fourth step; and
a sixth step of generating control data in order to optimize motion assistance control performance by the wearable motion assistance device (1) on the basis of an analysis result of the fifth step.

## Patentansprüche

**1.** Gerät zur Bewertung der Belastung des unteren Rückenbereichs (60), das eine tragbare Bewegungsunterstützungsvorrichtung (1) umfasst, die ausgelegt ist, durch Befestigen entweder beider Oberschenkelbereiche des Trägers oder des Bauchbereichs und Schulterbereichs des Trägers oder von beidem getragen zu werden, um Bewegungen des unteren Rückenbereichs des Trägers zu unterstützen, wobei das Gerät zur Bewertung der Belastung des unteren Rückenbereichs (60) Folgendes umfasst:

eine Rumpfwinkel-Messeinheit (61), die in der tragbaren Bewegungsunterstützungsvorrichtung (1) bereitgestellt und ausgelegt ist, kontinuierlich den Neigungswinkel des unteren Rückenbereichs des Trägers als Rumpfwinkel des Trägers zu messen;
**gekennzeichnet durch** eine Bandscheibendruckkraft-Schätzeinheit (62), die ausgelegt ist, kontinuierlich die Bandscheibendruckkraft, die auf die Bandscheiben des Trägers in die Bandscheibenverbindungsrichtung wirkt, basierend auf dem Gewicht eines Objekts, das vom Träger gehalten wird, der Körpergröße und dem Körpergewicht des Trägers, die extern eingegeben werden, und dem Rumpfwinkel, der von der Rumpfwinkel-Messeinheit (61) gemessen wird, zu schätzen;
eine Messeinheit für verstrichene Zeitabschnitte (63), die ausgelegt ist, die von der Bandscheibendruckkraft-Schätzeinheit (62) geschätzte Bandscheibendruckkraft gemäß eines Belastungsgrads in Abschnitte zu unterteilen und gleichzeitig die verstrichene Zeit für jeden der Abschnitte in chronologischer Reihenfolge zu messen; und
eine Einheit zur Vorhersage des Risikos für das Auftreten von Rückenschmerzen (64), die ausgelegt ist, das Risiko des Trägers vorherzusagen, ein Schmerzsymptom im unteren Rücken zu entwickeln, basierend auf dem Messverlauf der Messeinheit für verstrichene Zeitabschnitte (63) während der Arbeitszeit mit schwerer Muskelarbeit zum Handhaben des Objekts.

**2.** Gerät zur Bewertung der Belastung des unteren Rückenbereichs (60), das eine tragbare Bewegungsunterstützungsvorrichtung (1) nach Anspruch 1 umfasst und weiters eine Hüftgelenkswinkel-Messeinheit (32) umfasst, die in der Bewegungsunterstützungsvorrichtung (1) bereitgestellt und ausgelegt ist, kontinuierlich Bewegungswinkel beim Beugen und Strecken des rechten bzw. linken Hüftgelenks des Trägers als rechten und linken Hüftgelenkwinkel des Trägers zu messen,
wobei die Bandscheibendruckkraft-Schätzeinheit (62) ausgelegt ist, kontinuierlich die Bandscheibendruckkraft, die auf die Bandscheiben des Trägers in die Bandscheibenverbindungsrichtung wirkt, basierend auf einer Kombination aus dem Rumpfwinkel des Trägers und dem rechten und linken Hüftgelenkwinkel des Trägers zu schätzen.

**3.** Gerät zur Bewertung der Belastung des unteren Rückenbereichs (60), das eine tragbare Bewegungsunterstützungsvorrichtung (1) nach Anspruch 1 oder 2 umfasst, wobei die Einheit zur Vorhersage des Risikos für das Auftreten von Rückenschmerzen (64) ausgelegt ist, basierend auf einem Schätzverlauf der Bandscheibendruckkraft-Schätzeinheit (62) zu bestimmen, dass das Risiko des Trägers, das Schmerzsymptom im unteren Rücken zu entwickeln, hoch ist, wenn der Maximalwert der Bandscheibendruckkraft während der Arbeitszeit nahe einem bestimmten Schwellengrenzwert liegt.

4. Gerät zur Bewertung der Belastung des unteren Rückenbereichs (60), das eine tragbare Bewegungsunterstützungsvorrichtung (1) nach einem der Ansprüche 1 bis 3 umfasst, wobei die Einheit zur Vorhersage des Risikos für das Auftreten von Rückenschmerzen (64) ausgelegt ist, basierend auf dem Messverlauf der Rumpfwinkel-Messeinheit (61) zu bestimmen, dass das Risiko des Trägers, das Schmerzsymptom im unteren Rücken zu entwickeln, hoch ist, wenn der Rumpfwinkel des Trägers weiterhin innerhalb eines bestimmten Bereichs liegt, um eine halb hockende Haltung während der Arbeitszeit für eine bestimmte Zeitdauer einzunehmen.

5. Gerät zur Bewertung der Belastung des unteren Rückenbereichs (60), das eine tragbare Bewegungsunterstützungsvorrichtung (1) nach einem der Ansprüche 1 bis 4 umfasst, wobei die Einheit zur Vorhersage des Risikos für das Auftreten von Rückenschmerzen (64) ausgelegt ist, einen Durchschnittswert der Belastung, die während der Arbeitszeit auf den unteren Rückenbereich des Trägers wirkt, basierend auf dem Messverlauf der Bandscheibendruckkraft-Schätzeinheit (62) sowie der Messeinheit für verstrichene Zeitabschnitte (63) während der Arbeitszeit zu berechnen und zu bestimmen, dass das Risiko des Trägers, das Schmerzsymptom im unteren Rücken zu entwickeln, hoch ist, wenn der Durchschnittswert gleich wie oder größer als ein bestimmter Schwellenwert wird.

6. Gerät zur Bewertung der Belastung des unteren Rückenbereichs (60), das eine tragbare Bewegungsunterstützungsvorrichtung (1) nach einem der Ansprüche 1 bis 5 umfasst, wobei die Einheit zur Vorhersage des Risikos für das Auftreten von Rückenschmerzen (64) ausgelegt ist, das Risiko des Trägers, das Schmerzsymptom im unteren Rücken zu entwickeln gemäß einer Beziehung zwischen dem Maximalwert des Rumpfwinkels des Trägers und dem Gewicht des Objekts während der Arbeitszeit basierend auf dem Messverlauf der Rumpfwinkel-Messeinheit (61) vorherzusagen.

7. Gerät zur Bewertung der Belastung des unteren Rückenbereichs (60), das eine tragbare Bewegungsunterstützungsvorrichtung (1) nach einem der Ansprüche 1 bis 6 umfasst, wobei die Einheit zur Vorhersage des Risikos für das Auftreten von Rückenschmerzen (64) ausgelegt ist, basierend auf dem Messverlauf der Messeinheit für verstrichene Zeitabschnitte (63) während der Arbeitszeit zu bestimmen, dass das Risiko des Trägers, das Schmerzsymptom im unteren Rücken zu entwickeln, hoch ist, wenn die akkumulierte Zeit, in der der Belastungsgrad der Bandscheibendruckkraft während der Arbeitszeit in einen Abschnitt gehört, der gleich wie oder höher als ein spezifizierter Grad ist, gleich wie oder länger als eine bestimmte Zeitdauer ist.

8. Gerät zur Bewertung der Belastung des unteren Rückenbereichs (60), das eine tragbare Bewegungsunterstützungsvorrichtung (1) nach einem der Ansprüche 1 bis 7 umfasst und weiters Folgendes umfasst:

   eine Arbeitsbelastungsanalyseeinheit (70), die ausgelegt ist, eine Arbeitsbelastung zu analysieren, um die Belastung auf dem unteren Rückenbereich und dem Endoskelettsystem des Trägers in Verbindung mit der schweren Muskelarbeit basierend auf einem vorhergesagten Ergebnis der Einheit zur Vorhersage des Risikos für das Auftreten von Rückenschmerzen (64) zu verringern; und
   eine Optimierungssteuerungsdaten-Generierungseinheit (71), die ausgelegt ist, Steuerungsdaten zu generieren, um die Bewegungsunterstützungssteuerungsleistung der tragbaren Bewegungsunterstützungsvorrichtung (1) basierend auf einem Analyseergebnis der Arbeitsbelastungsanalyseeinheit (70) zu optimieren.

9. Gerät zur Bewertung der Belastung des unteren Rückenbereichs (60), das eine tragbare Bewegungsunterstützungsvorrichtung (1) nach einem der Ansprüche 1 bis 8 umfasst, wobei die tragbare Bewegungsunterstützungsvorrichtung (1) Folgendes umfasst:

   einen Antriebsmechanismus (31), der ausgelegt ist, Oberschenkelbereichsbefestigungseinheiten (20) oder einen Rahmen für den unteren Rücken (2) durch Erzeugen eines Antriebsdrehmoments anzutreiben, um jedes aus dem Rahmen für den unteren Rücken (2), der am unteren Rücken des Trägers angebracht ist und mit dessen rechter und linker Seite verbunden werden kann, und den Oberschenkelbereichsbefestigungseinheiten (20), die am rechten und linken Oberschenkelbereich des Trägers befestigt sind und jeweils mit der Außenseite der Oberschenkelbereiche verbunden werden kann, relativ rotatorisch anzutreiben;
   eine Hüftgelenkswinkel-Messeinheit (32), die im Antriebsmechanismus (31) bereitgestellt ist und Rotationswinkel zwischen dem Rahmen für den unteren Rücken (2) und den Oberschenkelbereichsbefestigungseinheiten (20) als rechten bzw. linken Hüftgelenkswinkel des Trägers detektiert; und
eine Steuereinheit (33), die eine Bewegung und die Haltung des Trägers basierend auf dem rechten und linken Hüftgelenkswinkel des Trägers laut der Hüftgelenkswinkel-Messeinheit (32) beurteilt und bewirkt, dass der Antriebsmechanismus (31) ein Antriebsdrehmoment gemäß einem Ergebnis der Beurteilung generiert.

10. Gerät zur Bewertung der Belastung des unteren Rückenbereichs (60), das eine tragbare Bewegungsunterstützungsvorrichtung (1) nach Anspruch 9 umfasst und weiters eine Biosignal-Detektionseinheit (34) umfasst, die ausgelegt ist, um ein Biosignal eines Musculus rectus femoris und eines Musculus gluteus maximus in Verbindung mit Bewegungen des linken Oberschenkels und rechten Oberschenkels des Trägers und ein Biosignal des Musculus latissimus dorsi zu detektieren, wobei die Steuereinheit (33) ausgelegt ist, um die Bewegung und Haltung des Trägers basierend auf den Biosignalen, die aus der Biosignal-Detektion ausgegeben werden, und den rechten und linken Hüftgelenkwinkel des Trägers zu beurteilen und zu bewirken, dass der Antriebsmechanismus (31) ein Antriebsdrehmoment gemäß einem Ergebnis der Beurteilung generiert.

11. Verfahren zur Bewertung der Belastung des unteren Rückenbereichs unter Verwendung einer tragbaren Bewegungsunterstützungsvorrichtung (1), die durch Befestigen entweder beider Oberschenkelbereiche des Trägers oder des Bauchbereichs und Schulterbereichs des Trägers oder von beidem getragen wird, um Bewegungen des unteren Rückenbereichs des Trägers zu unterstützen,
wobei das Verfahren zur Bewertung der Belastung des unteren Rückenbereichs Folgendes umfasst:

einen ersten Schritt des kontinuierlichen Messens des Neigungswinkels des unteren Rückenbereichs des Trägers, der die tragbare Bewegungsunterstützungsvorrichtung (1) trägt, als einen Rumpfwinkel des Trägers;
einen zweiten Schritt des kontinuierlichen Schätzens der Bandscheibendruckkraft, die auf die Bandscheiben des Trägers in die Bandscheibenverbindungsrichtung wirkt, basierend auf dem Gewicht eines Objekts, das vom Träger gehalten wird, der Körpergröße und dem Körpergewicht des Trägers, die extern eingegeben werden, und dem im ersten Schritt gemessenen Rumpfwinkel;
einen dritten Schritt des Unterteilens der im zweiten Schritt geschätzten Bandscheibendruckkraft in Abschnitte gemäß einem Belastungsgrad und des gleichzeitigen Messens der verstrichenen Zeit für jeden der Abschnitte in chronologischer Reihenfolge; und
einen vierten Schritt des Vorhersagens des Risikos des Trägers, ein Schmerzsymptom im unteren Rücken zu entwickeln, basierend auf dem Messverlauf im dritten Schritt während der Arbeitszeit mit schwerer Muskelarbeit zum Handhaben des Objekts.

12. Verfahren zur Bewertung der Belastung des unteren Rückenbereichs unter Verwendung einer tragbaren Bewegungsunterstützungsvorrichtung (1) nach Anspruch 11,

wobei im ersten Schritt Bewegungswinkel beim Beugen und Strecken des rechten bzw. linken Hüftgelenks des Trägers kontinuierlich als rechter und linker Hüftgelenkwinkel des Trägers gemessen werden,
wobei im zweiten Schritt die Bandscheibendruckkraft, die auf die Bandscheiben des Trägers in die Bandscheibenverbindungsrichtung wirkt, kontinuierlich basierend auf einer Kombination aus dem Rumpfwinkel des Trägers und dem rechten und linken Hüftgelenkwinkel des Trägers geschätzt wird.

13. Verfahren zur Bewertung der Belastung des unteren Rückenbereichs unter Verwendung einer tragbaren Bewegungsunterstützungsvorrichtung (1) nach Anspruch 11 oder 12,
wobei im vierten Schritt basierend auf dem Schätzverlauf des dritten Schritts bestimmt wird, dass das Risiko des Trägers, das Schmerzsymptom im unteren Rücken zu entwickeln, hoch ist, wenn der Maximalwert der Bandscheibendruckkraft während der Arbeitszeit nahe einem bestimmten Schwellengrenzwert liegt.

14. Verfahren zur Bewertung der Belastung des unteren Rückenbereichs unter Verwendung einer Bewegungsunterstützungsvorrichtung (1) nach einem der Ansprüche 11 bis 13,
wobei im vierten Schritt basierend auf dem Messverlauf des ersten Schritts bestimmt wird, dass das Risiko des Trägers, das Schmerzsymptom im unteren Rücken zu entwickeln, hoch ist, wenn der Rumpfwinkel des Trägers weiterhin innerhalb eines bestimmten Bereichs liegt, um eine halb hockende Haltung während der Arbeitszeit für eine bestimmte Zeitdauer einzunehmen.

15. Verfahren zur Bewertung der Belastung des unteren Rückenbereichs unter Verwendung einer Bewegungsunterstützungsvorrichtung (1) nach einem der Ansprüche 11 bis 14,
wobei im vierten Schritt ein Durchschnittswert der Belastung, die während der Arbeitszeit auf den unteren Rückenbereich des Trägers wirkt, basierend auf dem Messverlauf des zweiten Schritts und des dritten Schritts berechnet wird und bestimmt wird, dass das Risiko des Trägers, das Schmerzsymptom im unteren Rücken zu entwickeln, hoch ist, wenn der Durchschnittswert gleich wie oder größer als ein bestimmter Schwellenwert wird.

16. Verfahren zur Bewertung der Belastung des unteren Rückenbereichs unter Verwendung einer Bewegungsunterstützungsvorrichtung (1) nach einem der Ansprüche 11 bis 15,
wobei im vierten Schritt das Risiko des Trägers, das Schmerzsymptom im unteren Rücken zu entwickeln, gemäß einer Beziehung zwischen dem Maximalwert des Rumpfwinkels des Trägers und dem Gewicht des Objekts während der Arbeitszeit basierend auf dem Messverlauf des ersten Schritts vorhergesagt wird.

17. Verfahren zur Bewertung der Belastung des unteren Rückenbereichs unter Verwendung einer Bewegungsunterstützungsvorrichtung (1) nach einem der Ansprüche **11** bis 16,
wobei im vierten Schritt basierend auf dem Messverlauf des dritten Schritts während der Arbeitszeit bestimmt wird, dass das Risiko des Trägers, das Schmerzsymptom im unteren Rücken zu entwickeln, hoch ist, wenn die akkumulierte Zeit, in der der Belastungsgrad der Bandscheibendruckkraft während der Arbeitszeit in einen Abschnitt gehört, der gleich wie oder höher als ein bestimmter Grad ist, gleich wie oder länger als eine bestimmte Zeitdauer ist.

18. Verfahren zur Bewertung der Belastung des unteren Rückenbereichs unter Verwendung einer Bewegungsunterstützungsvorrichtung (1) nach einem der Ansprüche **11** bis 17, das weiters Folgendes umfasst:

einen fünften Schritt des Analysierens der Arbeitsbelastung, um die Belastung auf dem unteren Rückenbereich und dem Endoskelettsystem des Trägers in Verbindung mit der schweren Muskelarbeit basierend auf einem vorhergesagten Ergebnis des vierten Schritts zu verringern; und
einen sechsten Schritt des Generierens von Steuerungsdaten, um die Bewegungsunterstützungssteuerungsleistung der tragbaren Bewegungsunterstützungsvorrichtung (1) basierend auf einem Analyseergebnis des fünften Schritts zu optimieren.

**Revendications**

1. Appareil d'évaluation de charge d'une partie de bas du dos (60) comprenant un dispositif d'assistance au mouvement pouvant être porté sur soi (1) qui est configuré pour être porté en étant fixé soit sur les deux parties fémorales d'un porteur, soit sur la partie abdominale et les épaules du porteur, ou les deux, afin d'assister les mouvements d'une partie de bas du dos du porteur, l'appareil d'évacuation de charge d'une partie de bas du dos (60) comprenant :

une unité de mesure d'angle de tronc (61) qui est prévue dans le dispositif d'assistance au mouvement pouvant être porté sur soi (1) et est configurée pour mesurer en continu un angle d'inclinaison de la partie de bas du dos du porteur sous la forme d'un angle de tronc du porteur ;
**caractérisé par** une unité d'estimation de force de pression de disques intervertébraux (62) qui est configurée pour estimer en continu une force de pression de disques intervertébraux exercée sur les disques intervertébraux du porteur dans une direction de liaison entre les corps vertébraux sur la base d'un poids d'un objet tenu par le porteur et d'une hauteur de corps et d'un poids corporel du porteur qui sont saisis en externe, et de l'angle de tronc mesuré par l'unité de mesure d'angle de tronc (61) ;
une unité de mesure de durée écoulée par sections (63) qui est configurée pour diviser la force de pression de disques intervertébraux estimée par l'unité d'estimation de force de pression de disques intervertébraux (62) en sections selon un niveau de charge et est configurée pour, en même temps, mesurer la durée écoulée pour chacune des sections dans l'ordre chronologique ; et
une unité de prédiction de risque d'occurrence de douleur dans le bas du dos (64) qui est configurée pour prédire le risque, pour le porteur, de développer un symptôme de douleur dans le bas du dos sur la base d'un historique de mesure de l'unité de mesure de durée écoulée par sections (63) pendant un travail musculaire intense pour manipuler l'objet.

2. Appareil d'évaluation de charge d'une partie de bas du dos (60) comprenant le dispositif d'assistance au mouvement pouvant être porté sur soi (1) selon la revendication 1, comprenant en outre une unité de mesure d'angle d'articulation de la hanche (32) qui est prévue dans le dispositif d'assistance au mouvement pouvant être porté sur soi (1) et est configurée pour mesurer en continu les angles de mouvement lors de la flexion et de l'étirement des articulations de hanche droit et gauche du porteur, respectivement, sous la forme d'angles d'articulation de hanche droite et gauche du porteur,

EP 4 026 665 B1

dans lequel l'unité d'estimation de force de pression de disques intervertébraux (62) est configurée pour estimer en continu la force de pression de disques intervertébraux exercée sur les disques intervertébraux du porteur dans la direction de liaison entre les corps vertébraux sur la base d'une combinaison de l'angle de tronc du porteur et des angles d'articulation de hanche droite et gauche du porteur.

3. Appareil d'évaluation de charge d'une partie de bas du dos (60) comprenant le dispositif d'assistance au mouvement pouvant être porté sur soi (1) selon la revendication 1 ou 2, dans lequel l'unité de prédiction de disque d'occurrence de douleur dans le bas du dos (64) est configurée pour déterminer, sur la base d'un historique d'estimation de l'unité d'estimation de force de pression de disques intervertébraux (62), que le risque, pour le porteur, de développer un symptôme de douleur dans le bas du dos est élevé lorsqu'une valeur maximale de la force de pression de disques intervertébraux pendant le temps de travail est proche d'une valeur limite de seuil spécifiée.

4. Appareil d'évaluation de charge d'une partie de bas du dos (60) comprenant le dispositif d'assistance au mouvement pouvant être porté sur soi (1) selon l'une quelconque des revendications 1 à 3, dans lequel l'unité de prédiction de risque d'occurrence de douleur dans le bas du dos (64) est configurée pour déterminer, sur la base d'un historique de mesure de l'unité de mesure d'angle de tronc (61), que le risque, pour le porteur, de développer le symptôme de douleur dans le bas du dos est élevé lorsque l'angle de tronc du porteur continue à se trouver dans des limites spécifiées pour adopter une posture semi-accroupie pendant le temps de travail pendant une durée spécifiée.

5. Appareil d'évaluation de charge d'une partie de bas du dos (60) comprenant le dispositif d'assistance au mouvement pouvant être porté sur soi (1) selon l'une quelconque des revendications 1 à 4, dans lequel l'unité de prédiction de risque d'occurrence de douleur dans le bas du dos (64) est configurée pour calculer une valeur moyenne de la charge imposée sur la partie de bas du dos du porteur pendant le temps de travail sur la base de chaque historique de mesure de l'unité d'estimation de force de pression de disques intervertébraux (62) et de l'unité de mesure de durée écoulée par sections (63) pendant le temps de travail et pour déterminer que le risque, pour le porteur, de développer le symptôme de douleur dans le bas du dos est élevé lorsque la valeur moyenne devient égale ou supérieure à une valeur de seuil spécifiée.

6. Appareil d'évaluation de charge d'une partie de bas du dos (60) comprenant le dispositif d'assistance au mouvement pouvant être porté sur soi (1) selon l'une quelconque des revendications 1 à 5, dans lequel l'unité de prédiction d'occurrence de douleur dans le bas du dos (64) est configurée pour prédire le risque, pour le porteur, de développer le symptôme de douleur dans le bas du dos selon une relation entre une valeur maximale de l'angle de tronc du porteur et un poids de l'objet pendant le temps de travail sur la base de l'historique de mesure de l'unité de mesure d'angle de tronc (61).

7. Appareil d'évaluation de charge d'une partie de bas du dos (60) comprenant le dispositif d'assistance au mouvement pouvant être porté sur soi (1) selon l'une quelconque des revendications 1 à 6, dans lequel l'unité de prédiction de risque d'occurrence de douleur dans le bas du dos (64) est configurée pour déterminer, sur la base de l'historique de mesure de l'unité de mesure de durée écoulée par sections (63) pendant le temps de travail, que le risque, pour le porteur, de développer le symptôme de douleur dans le bas du dos est élevé lorsque la durée cumulée pendant laquelle le niveau de charge de la force de pression de disques intervertébraux pendant le temps de travail appartient à une section égale ou supérieure à un niveau spécifié est égale ou supérieure à une période spécifiée.

8. Appareil d'évaluation de charge d'une partie de bas du dos (60) comprenant le dispositif d'assistance au mouvement pouvant être porté sur soi (1) selon l'une quelconque des revendications 1 à 7, comprenant en outre :

une unité d'analyse de charge de travail (70) qui est configurée pour analyser une charge de travail afin de réduire la charge sur la partie de bas du dos et un système d'endosquelette du porteur en lien avec le travail musculaire intense sur la base d'un résultat prédit de l'unité de prédiction de risque d'occurrence de douleur dans le bas du dos (64) ; et
une unité de génération de données de contrôle d'optimisation (71) qui est configurée pour générer des données de contrôle afin d'optimiser les performances de contrôle d'assistance au mouvement par le dispositif d'assistance au mouvement pouvant être porté sur soi (1) sur la base d'un résultat d'analyse de l'unité d'analyse de charge de travail (70).

9. Appareil d'évaluation de charge d'une partie de bas du dos (60) comprenant le dispositif d'assistance au mouvement pouvant être porté sur soi (1) selon l'une quelconque des revendications 1 à 8, dans lequel le dispositif d'assistance au mouvement pouvant être porté sur soi (1) comporte :

un mécanisme d'entraînement (31) qui est configuré pour entraîner des unités de fixation sur les parties fémorales (20) ou une armature de bas du dos (2) en générant un couple d'entraînement afin d'entraîner relativement en rotation chaque armature de bas du dos (2) qui est configurée pour être montée sur le bas du dos du porteur et qui peut être reliée à ses côtés droit et gauche, et les unités de fixation sur les parties fémorales (20) qui sont configurées pour être fixées sur les parties fémorales du porteur et qui peuvent être reliées à un extérieur de chacune des parties fémorales ;

une unité de mesure d'angle d'articulation de hanche (32) qui est prévue dans le mécanisme d'entraînement (31) et qui détecte les angles de rotation entre l'armature de bas du dos (2) et les unités de fixation sur les parties fémorales (20) respectivement sous la forme d'angles d'articulation de hanche droite et gauche du porteur ; et

une unité de commande (33) qui juge un mouvement et une posture du porteur sur la base des angles d'articulation de hanche droite et gauche du porteur par l'unité de mesure d'angle d'articulation de hanche (32) et permet au mécanisme d'entraînement (31) de générer un couple d'entraînement selon un résultat du jugement.

10. Appareil d'évaluation de charge d'une partie de bas du dos (60) comprenant le dispositif d'assistance au mouvement pouvant être porté sur soi (1) selon la revendication 9, comprenant en outre une unité de détection de biosignal (34) qui est configurée pour détecter un biosignal d'un muscle droit antérieur de la cuisse et d'un muscle grand fessier associés aux mouvements de la cuisse gauche et de la cuisse droite du porteur et un biosignal d'un muscle grand dorsal du dos du porteur,

dans lequel l'unité de commande (33) est configurée pour juger le mouvement et la posture du porteur sur la base des biosignaux délivrés par la détection de biosignal et des angles d'articulation de hanche droite et gauche du porteur et permet au mécanisme d'entraînement (31) de générer un couple d'entraînement selon un résultat du jugement.

11. Procédé d'évaluation de charge de partie de bas du dos à l'aide d'un dispositif d'assistance au mouvement pouvant être porté sur soi (1) porté en fixant soit les deux parties fémorales d'un porteur, soit la partie abdominale et les épaules du porteur, ou les deux, afin d'assister les mouvements d'une partie de bas du dos du porteur, le procédé d'évaluation de charge de bas du dos comprenant :

une première étape qui consiste à mesurer en continu un angle d'inclinaison de la partie de bas du dos du porteur qui porte le dispositif d'assistance au mouvement pouvant être porté sur soi (1) sous la forme d'un angle de tronc du porteur ;

une deuxième étape qui consiste à estimer une force de pression de disques intervertébraux exercée sur les disques intervertébraux du porteur dans une direction de liaison entre les corps vertébraux sur la base d'un poids d'un objet tenu par le porteur et d'une hauteur de corps et d'un poids corporel du porteur qui sont saisis en externe, et de l'angle de tronc mesuré à la première étape ;

une troisième étape qui consiste à diviser la force de pression de disques intervertébraux estimée à la deuxième étape en sections selon un niveau de charge et, en même temps, pour mesurer la durée écoulée pour chacune des sections dans l'ordre chronologique ; et

une quatrième étape de prédiction du risque, pour le porteur, de développer un symptôme de douleur dans le bas du dos sur la base d'un historique de mesure à la troisième étape pendant un travail musculaire intense pour manipuler l'objet.

12. Procédé d'évaluation de charge de partie de bas du dos à l'aide du dispositif d'assistance au mouvement pouvant être porté sur soi (1) selon la revendication 11,

dans lequel, à la première étape, les angles de mouvement lors de la flexion et de l'étirement des articulations de hanche droite et gauche du porteur sont mesurés en continu, respectivement, sous la forme d'angles d'articulation de droite et de gauche du porteur,

dans lequel, à la deuxième étape, la force de pression de disques intervertébraux exercée sur les disques intervertébraux du porteur dans la direction de liaison entre les corps vertébraux est estimée en continu sur la base d'une combinaison de l'angle de tronc du porteur et des angles d'articulation de gauche droite et gauche du porteur.

13. Procédé d'évaluation de charge de partie de bas du dos à l'aide du dispositif d'assistance au mouvement pouvant être porté sur soi (1) selon la revendication 11 ou 12, dans lequel, à la quatrième étape, il est déterminé, sur la base d'un historique d'estimation de la troisième étape, que le risque, pour le porteur, de développer le symptôme de douleur dans le bas du dos est élevé lorsqu'une valeur maximale de la force de pression de disques intervertébraux pendant le temps de travail est proche d'une valeur limite de seuil spécifiée.

**14.** Procédé d'évaluation de charge de partie de bas du dos à l'aide du dispositif d'assistance au mouvement pouvant être porté sur soi (1) selon l'une quelconque des revendications 11 à 13,
dans lequel, à la quatrième étape, il est déterminé, sur la base d'un historique de mesure de la première étape, que le risque, pour le porteur, de développer le symptôme de douleur dans le bas du dos est élevé lorsque l'angle de tronc du porteur continue à se trouver dans des limites spécifiées pour adopter une posture semi-accroupie pendant le temps de travail pendant une durée spécifiée.

**15.** Procédé d'évaluation de charge de partie de bas du dos à l'aide du dispositif d'assistance au mouvement pouvant être porté sur soi (1) selon l'une quelconque des revendications 11 à 14,
dans lequel, à la quatrième étape, une valeur moyenne de la charge imposée sur la partie de bas du dos du porteur pendant le temps de travail est calculée sur la base de chaque historique de mesure de la deuxième étape et de la troisième étape, et il est déterminé que le risque, pour le porteur, de développer le symptôme de douleur dans le bas du dos est élevé lorsque la valeur moyenne devient égale ou supérieure à une valeur de seuil spécifiée.

**16.** Procédé d'évaluation de charge de partie de bas du dos à l'aide du dispositif d'assistance au mouvement pouvant être porté sur soi (1) selon l'une quelconque des revendications 11 à 15,
dans lequel, à la quatrième étape, le risque, pour le porteur, de développer le symptôme de douleur dans le bas du dos selon une relation entre une valeur maximale de l'angle de tronc du porteur et un poids de l'objet pendant le temps de travail est prédit sur la base de l'historique de mesure de la première étape.

**17.** Procédé d'évaluation de charge de partie de bas du dos à l'aide du dispositif d'assistance au mouvement pouvant être porté sur soi (1) selon l'une quelconque des revendications 11 à 16,
dans lequel, à la quatrième étape, il est déterminé, sur la base de l'historique de mesure de la troisième étape pendant le temps de travail, que le risque, pour le porteur, de développer le symptôme de douleur dans le bas du dos est élevé lorsque la durée cumulée pendant laquelle le niveau de charge de la force de pression de disques intervertébraux pendant le temps de travail appartient à une section égale ou supérieure à un niveau spécifié est égale ou supérieure à une période spécifiée.

**18.** Procédé d'évaluation de charge de partie de bas du dos à l'aide du dispositif d'assistance au mouvement pouvant être porté sur soi (1) selon l'une quelconque des revendications 11 à 17, comprenant en outre :

une cinquième étape d'analyse d'une charge de travail afin de réduire la charge sur la partie de bas du dos et un système d'endosquelette du porteur en lien avec le travail musculaire intense sur la base d'un résultat prédit de la quatrième étape ; et
une sixième étape qui consiste à générer des données de contrôle afin d'optimiser les performances de contrôle d'assistance au mouvement par le dispositif d'assistance au mouvement pouvant être porté sur soi (1) sur la base d'un résultat d'analyse de la cinquième étape.

FIG. 1

(A)

(B)

FIG. 2

(B)

(A)

FIG. 3

(A)

1

(B)

23

FIG. 4

(A)

1

(B)

1

EP 4 026 665 B1

## FIG. 5

# FIG. 6

# FIG. 7

THORACIC VERTEBRA
INTERVERTEBRAL JOINTS

ROTATION +
LATEROFLECTION +
BENDING AND
STRETCHING -

LUMBAR VERTEBRA
INTERVERTEBRAL JOINTS

BENDING AND
STRETCHING +
LATEROFLECTION-
ROTATION -

$L_1$-$L_2$
$L_2$-$L_3$ } 5~10%
$L_3$-$L_4$
$L_4$-$L_5$  20~25%
$L_5$-$S_1$  70~75%

FIG. 8

EP 4 026 665 B1

(A)

(B)

# FIG. 9

33  CONTROL APPARATUS

60  LOWER BACK PART LOAD
EVALUATION APPARATUS

| | |
|---|---|
| TRUNK ANGLE MEASURING UNIT | 61 |
| INTERVERTEBRAL DISK PRESSURE FORCE ESTIMATION UNIT | 62 |
| SECTION ELAPSED TIME MEASURING UNIT | 63 |
| LOWER BACK PAIN OCCURRENCE RISK FORECASTING UNIT | 64 |
| WORKLOAD ANALYSIS UNIT | 70 |
| OPTIMIZATION CONTROL DATA GENERATION UNIT | 71 |

# FIG. 10

## (A)

X-axis: HELD HEAVY LOAD WEIGHT (0kg, 5kg, 10kg, 15kg, 20kg, 25kg)

Y-axis: INTERVERTEBRAL DISK PRESSURE FORCE [N] (0 to 4000)

## (B)

LOAD LEVEL CORRESPONDENCE TABLE

| | INTERVERTEBRAL DISK PRESSURE FORCE Fc[N] | | |
|---|---|---|---|
| LOAD LEVEL 1 | 1800~ | LOAD CORRESPONDING TO 5 kg OR LESS | |
| LOAD LEVEL 2 | 2200~ | LOAD CORRESPONDING TO 10 kg | LOW LOAD |
| LOAD LEVEL 3 | 2600~ | LOAD CORRESPONDING TO 15 kg | MEDIUM LOAD |
| LOAD LEVEL 4 | 3000~ | LOAD CORRESPONDING TO 20 kg | HIGH LOAD |
| LOAD LEVEL 5 | 3400~ | LOAD CORRESPONDING TO 25 kg OR MORE | |

## FIG. 11

- — ·· — WORK TO BE AVOIDED
- ········ HIGH-LOAD WORK
- — · — MEDIUM-LOAD WORK
- — — — LOW-LOAD WORK
- ——— IDEAL WORK

Y-axis: HANDLED WEIGHT [KG]

X-axis: WORK POSTURE [DEG]

EP 4 026 665 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012183291 A
- JP 2015134149 A
- JP 2017136313 A
- WO 2019161232 A1